# EUROPEAN PATENT SPECIFICATION

(11) **EP 0 577 303 B1**
(45) Date of publication and mention of the grant of the patent: **01.10.1997**
(21) Application number: 93304817.5
(22) Date of filing: 21.06.1993
(51) Int. Cl.: C07H 19/04, A61K 31/70

(54) **Stereoselective glycosylation process**
Verfahren zur Stereoselektivglycosylierung
Procédé de glycosylation steréoséléctive

(30) Priority: 22.06.1992 US 902313; 07.04.1993 US 44345; 22.06.1992 US 902312; 07.04.1993 US 44343; 22.06.1992 US 902302; 07.04.1993 US 44309; 22.06.1992 US 902112; 07.04.1993 US 44312; 22.06.1992 US 902135; 07.04.1993 US 44315; 22.06.1992 US 902150; 07.04.1993 US 44996
(43) Date of publication of application: 05.01.1994
(73) Proprietor: ELI LILLY AND COMPANY, Indianapolis, Indiana 46285 (US)
(72) Inventor: Chou, Ta-Sen, Indianapolis, Indiana 46236 (US); Poteet, Laurie Michelle, Zionsville, Indiana 46077 (US); Kjell, Douglas Patton, West Lafayette, Indiana 47906 (US); Grossman, Cora Sue, Indianapolis, Indiana 46250 (US); Hertel, Larry Wayne, Indianapolis, Indiana 46239 (US); Holmes, Richard Elmer, Indianapolis, Indiana 46226 (US); Jones, Charles David, Indianapolis, Indiana 46227 (US); Mabry, Thomas Edward, Indianapolis, Indiana 46227 (US)
(74) Representative: Tapping, Kenneth George

(56) References cited:
- EP-A- 0 349 928
- EP-A- 0 350 292
- EP-A- 0 428 109
- WO-A-92/01700
- JOURNAL OF MEDICINAL CHEMISTRY vol. 33, no. 8, 1993, WASHINGTON US pages 2150 - 7 R.Z.STERZYCKI ET AL. 'Synthesis and Anti-HIV Activity of Several 2'-Fluoro-Containing Pyrimidine Nucleosides.'
- R.W.ALDER ET AL. 'Mechanism in Organic Chemistry.' 1978 , WILEY - INTERSCIENCE , SURREY, ENGLAND

## Description

This invention relates to a stereoselective glycosylation process for preparing 2'-deoxyfluoronucleosides and to intermediates of that process.

The continued interest in the synthesis of 2'-deoxyfluoronucleosides and their analogues is predicated on their successful use as therapeutic agents for treating viral and cancerous diseases. A compound of particular interest is gemcitabine; see European Patent Specification No. 211354 and U.S. Patent No. 4,526,988. Since these compounds are β nucleosides, there is a need to provide such compounds in high yield.

A critical step in the synthesis of 2'-deoxyfluoronucleosides is the condensation or glycosylation of the nucleobase and carbohydrate to form a N-glycoside bond. However, processes for synthesis of 2'-deoxynucleosides are typically non-stereoselective forming mixtures of α and β nucleosides. For instance, U.S. Patent 4,526,988 did not stereoselectively produce 2-deoxy-2,2-difluoro-β-nucleosides but instead produced a 4:1 α to β anomer ratio of 2-deoxy-2,2-difluoronucleoside. Even optimizing the protecting groups could not increase the α to β ratio beyond 1:1; see U.S. Patent No. 4,965,374 which utilized benzoyl blocking groups on the carbohydrate.

According to the present invention there is provided a stereoselective glycosylation process for preparing a β anomer enriched nucleoside of the formula 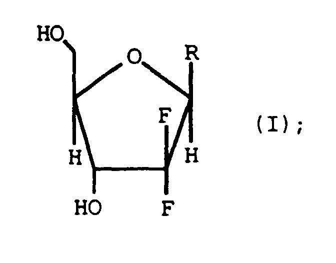 wherein R is a nucleobase selected from the group consisting of 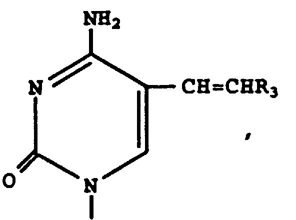 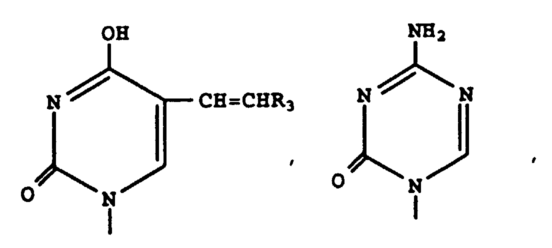 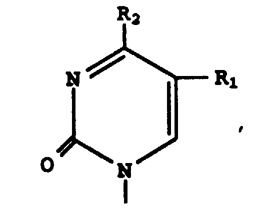 and 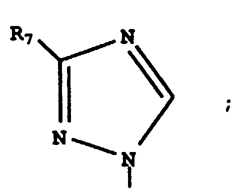 wherein R₁ is selected from the group consisting of hydrogen, alkyl, substituted alkyl and halo; R₂ is selected from the group consisting of hydroxy, halo, azido, primary amino and secondary amino; R₃ is selected from the group consisting of hydrogen, alkyl, and halo; R₇ is selected from the group consisting of hydrogen, halo, cyano, alkyl, alkoxy, alkoxycarbonyl, thioalkyl, thiocarboxamido and carboxamido, comprising the S_{N}2 nucleophilic displacement of a sulfonyloxy group (Y) from a α anomer enriched carbohydrate of the formula 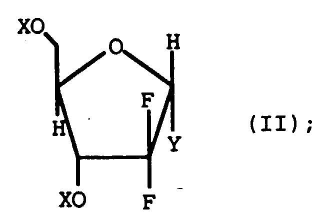 wherein X is independently selected from hydroxy protecting groups; with at least a molar equivalent of a nucleobase (R") selected from the group consisting of 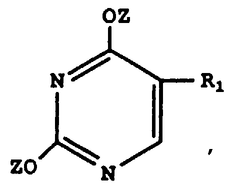 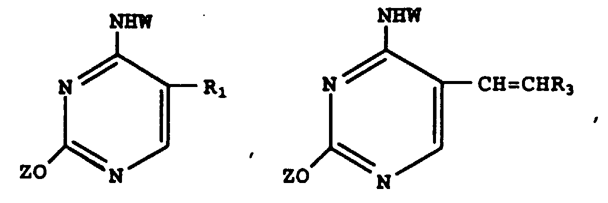 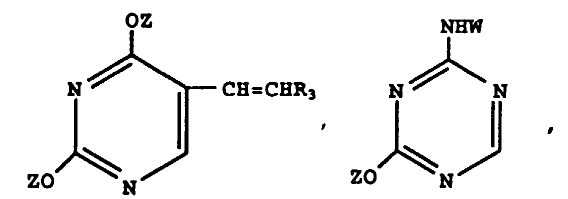 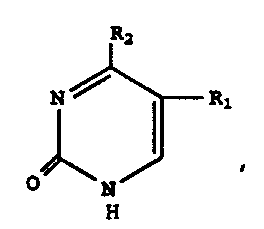 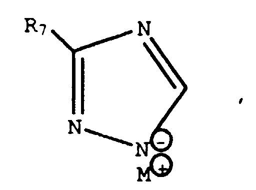 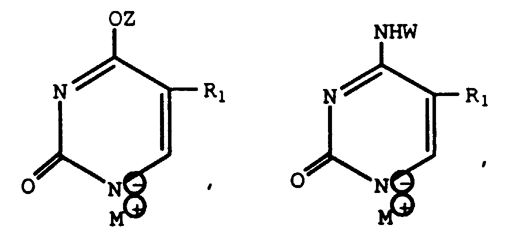 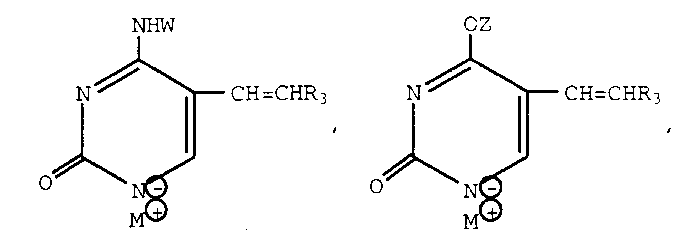 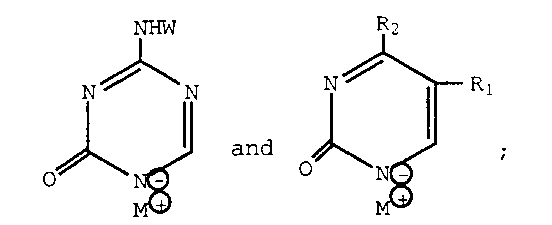 wherein R₁ through R₇ and Q are as defined above and; Z is a hydroxy protecting group; W is an amino protecting group; and M⁺ is a cation; and deblocking to form the compound of the formula (I).

Throughout this document, all temperatures are in degrees Celsius, all proportions, percentages and the like are in weight units and all mixtures are in volume units, except where otherwise indicated. Anomeric mixtures are expressed as a weight/weight ratio or as a percent. The term "lactol" alone or in combination refers to a 2-deoxy-2,2-difluoro-D-ribofuranose or 2-deoxy-2-fluoro-D-ribofuranose. The term "xylenes" alone or in combination refers to all isomers of xylene and mixtures thereof. The term "carbohydrate" alone or in combination refers to an activated lactol wherein the hydroxy group at the C-1 position has been replaced by a desirable leaving group. The term "halo" alone or in combination refers to chloro, iodo, fluoro and bromo. The term "alkyl" alone or in combination refers to straight, cyclic and branched chain aliphatic hydrocarbon groups which contain from 1 to 7 carbon atoms and preferably contain up to 4 carbon atoms, such as methyl, ethyl, n-propyl, isopropyl, n-butyl, t-butyl, n-pentyl, n-hexyl, 3-methylpentyl groups and the like or substituted straight, cyclic and branched chain aliphatic hydrocarbons, such as chloroethyl, 1,2-dichloroethyl, and the like. The term "alkoxy" alone or in combination refers to compounds of the general formula AO; wherein A is alkyl. The term "aryl" alone or in combination refers to carbocyclic or heterocyclic groups such as phenyl, naphthyl, thienyl and substituted derivatives thereof. The term "thioalkyl" alone or in combination refers to the general formula BS; wherein B is alkyl or hydrogen. The term "ester" alone or in combination refers to the general formula EOOC; wherein E is alkyl or aryl. The term "aromatic" alone or in combination refers to benzene-like structures containing (4n+2) delocalized π electrons. The terms "sulfonate" or "sulfonyloxy" alone or in combination refer to compounds of the general formula BSO₃; wherein B is alkyl, substituted alkyl, aryl or substituted aryl. The term "substituted" alone or in combination refers to a substitution by one or more of the groups selected from cyano, halo, carboalkoxy, toluoyl, nitro, alkoxy, alkyl, and dialkylamino. The phrase "anomer enriched" alone or in combination refers to an anomeric mixture wherein the ratio of a specified anomer is greater than 1:1 and includes substantially pure anomer. The term "concentrated" alone or in combination refers to a solution wherein the weight of carbohydrate dissolved in solvent is greater than 20 percent by weight per unit volume of solvent. For example, dissolving 100 grams of carbohydrate in 200 milliliters of solvent would form a 50 percent carbohydrate solution. The term "conjugate anion" refers to an anion of the general formula BSO₃-; wherein B is as defined above. The term "anomerization" alone or in combination refers to epimerization at the C-1 position of the ribofuranosyl derivative.

In accordance with the present glycosylation process, beta-anomer enriched 2'-deoxy-2',2'-difluoronucleosides and 2'-deoxy-2'-fluoronucleosides of formula (I) is prepared by reacting an α anomer enriched carbohydrate of formula (II) with at least a molar equivalent of a nucleobase (R") and deblocking the resulting nucleoside as shown below: 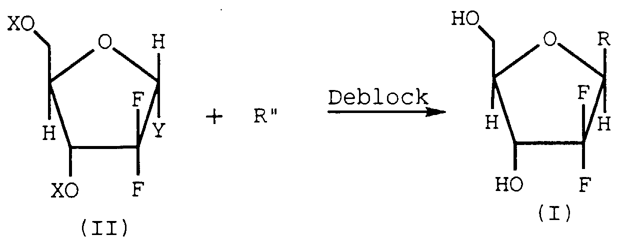 wherein Y, X, R" and R are as defined above. It is believed that the glycosylation reaction proceeds via S_{N}2 displacement. Therefore, the β anomer enriched nucleoside products of the present invention are stereoselectively derived from the reaction of the nucleobase with an α anomer enriched carbohydrate.

The lactol starting materials suitable for use in preparing the α anomer enriched carbohydrate of formula (II) used in the present glycosylation process are known in the art and are readily synthesized by standard procedures commonly employed by those of ordinary skill in the art. For example, U.S. Patent 4,526,988, incorporated herein by reference, teaches the synthesis of a 2,2-difluoro-2-deoxy-D-ribofuranoses intermediate of the formula 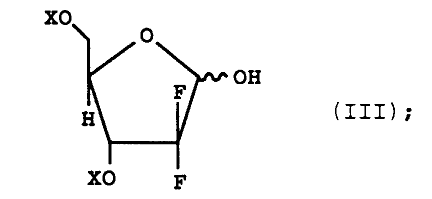 wherein X is a hydroxy protecting group. In addition, Reichman, et al., Carbohydr. Res., 42, 233 (1975) teaches the synthesis of a 2-deoxy-2-fluoro-D-ribofuranoses intermediate of the formula 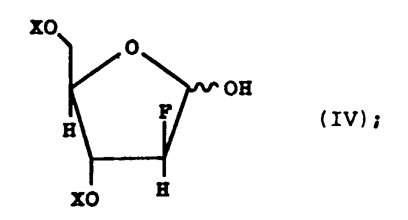 wherein X is a hydroxy protecting group. In a preferred embodiment of the present process, an aanomer enriched 2,2-difluoro-2-deoxy-D-ribofuranose-3,5-dibenzoate intermediate of formula (III) is employed.

A key feature of the present invention is the discovery that novel α anomer enriched carbohydrate intermediate of formula (III) or (IV) can be reacted under nucleophilic displacement conditions which favor inversion (i.e. S_{N}2 ) to provide the β anomer enriched nucleosides of formula (I).

To obtain an efficient reaction between the nucleobase and the α anomer enriched carbohydrate of formula (II), an appropriate leaving group (Y) must be stereoselectively attached to the lactol to activate the lactol and generate the α anomer enriched carbohydrate of formula (II). However, the leaving group selected depends on the nucleobase chosen and the glycosylation reaction conditions selected.

The α anomer enriched carbohydrate intermediate of formula (II) is preferably prepared by the teachings described in EP-A-576 229 and EP-A-577 302 EP-A-576 229 teaches a stereoselective process for preparing the α anomer enriched intermediate of formula (II) wherein T is fluoro by reacting a lactol of formula (III) with an amine base, having a pKa of from 8 to 20, in a low freezing inert solvent; adjusting the temperature of the reaction mixture from about -40°C to about -120°C; and adding a sulfonating reagent.

The amine base is preferably selected from the group consisting of triethylamine, triethylamine, tributylamine, dibutylamine, diethylmethylamine, dimethylethylamine, benzylmethylamine, N-methylmorpholine, tripropylamine, dipropylethylamine, N,N-dimethylbenzylamine, diisopropylethylamine, diethylamine, 1,8-diazabicyclo[5.4.0]undec-7-ene and 1,5-diazabicyclo[4.3.0]non-5-ene. The amount of base preferably employed ranges from about 1 molar equivalent to about 2 molar equivalents and more preferably from about 1.2 molar equivalents to about 1.5 molar equivalents.

The reaction is carried out in an inert solvent having a freezing point temperature preferably below -78°C. Preferred solvents are selected from the group consisting of dichloromethane, 1,2-dichloroethane, dichlorofluoromethane, acetone, toluene, anisole, chlorobenzene, and mixtures thereof.

The temperature of the solvent mixture is preferably below about -78°C. For example, a compound of formula III, where X is benzoyl, was added to dichloromethane and triethylamine at room temperature for 30 minutes. Next, the temperature of the reaction was lowered. An ¹⁹F NMR, was taken at various temperatures and showed that an increase in the α to β anomer ratio of the ionized lactol occurred as the temperature was lowered:

| Temperature | Alpha/Beta Ratio |
|---|---|
| 19°C | 2.0:1 |
| -3°C | 2.3:1 |
| -23°C | 2.5:1 |
| -43°C | 3.0:1 |
| -63°C | 3.6:1 |
| -83°C | 4.4:1 |

The ionized lactol is then trapped in solution at the lower temperature and higher α anomer ratio by adding a sulfonating reagent to form the α anomer enriched carbohydrate of formula (II). Thus, by appropriate selection of the temperature it is posssible to vary the α to β ratio of the carbohydrate intermediate starting material.

The leaving group (Y) is attached to the lactol by sulfonation. The sulfonating reagents are preferably selected from the group consisting of substituted and unsubstituted alkyl sulfonating halides,substituted and unsubstituted aryl sulfonating halides and alkyl sulfonic acid anhydrides and aryl sulfonic acid anhydrides such as, methanesulfonyl halide, ethanesulfonyl halide, 2-chloro-1-ethanesulfonyl halide, p-nitrobenezenesulfonyl halide, 2,4-dinitrobenzenesulfonyl halide, bromobenzenesulfonyl halide, dibromobenzenesulfonyl halide, benzenesulfonic acid anhydride, p-bromobenzenesulfonic acid anhydride and methanesulfonic acid anhydride and substituted and unsubstituted fluoro alkyl and fluoro aryl sulfonating halides and fluoro alkyl and fluoro aryl sulfonic acid anhydrides such as, trifluoromethanesulfonyl anhydride, trifluoromethanesulfonyl halide, 1,1,1-trifluoroethanesulfonyl halide, 1,1,1-trifluoroethanesulfonyl anhydride, octaflic (perfluorooctanesulfonic) acid halide, octaflic acid anhydride, nonaflic (nonafluorobutanesulfonic) acid halide and nonaflic acid anhydride, depending on the leaving group desired; more preferred is methanesulfonyl halide. α anomer enriched carbohydrate intermediates prepared from ionized lactols, especially carbohydrates containing trifluoromethane sulfonyloxy, are unstable at room temperature and therefore are preferably reacted with the nucleobase in-situ. Also, due to the reactivity of the sulfonating reagents, it may be desirable to carry out the glycosylation reaction in a batch or continuous mode for large scale operations.

α anomer enriched intermediates of formula (II) wherein T is hydrogen may be prepared in a similar manner, except a lactol of formula (IV) is used as the starting material..

EP-A-577 302 teaches a second stereoselective process for preparing the α anomer enriched intermediates of formula (II) wherein T is fluoro by treating a beta-anomer ribofuranosyl sulfonate of the formula 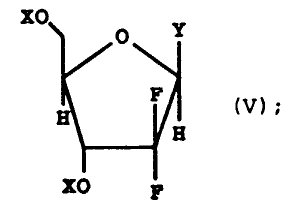 wherein Y is a sulfonate and each X is independently selected from hydroxy protecting groups with a source of a conjugate anion of a sulfonic acid, at elevated temperatures, in an inert solvent.

The conjugate anion of a sulfonic acid may be derived from a number of sources known to one of ordinary skill in the art. These include:
(a) neutralizing an alkyl or aryl sulfonic acid such as 1-methanesulfonic acid, p-methylbenzene sulfonic acid, ethanesulfonic acid, p-toluenesulfonic acid, benzenesulfonic acid, p-bromobenzenesulfonic acid and camphorsulfonic acid with an alkali metal base such as sodium hydroxide, sodium hydride, potassium hydroxide, potassium t-butoxide, sodium methoxide and the like;
(b) neutralizing the alkyl or aryl sulfonic acids above with an amine base such as triethylamine, trimethylamine, N,N-dimethylbenzylamine or N-methylmorpholine or with an aromatic nitrogenous base such as pyridine. Examples of conjugate anions of sulfonic acids prepared by this method include triethylammonium methanesulfonate, trimethylammonium methanesulfonate, N,N-dimethylbenzylammonium methanesulfonate, pyridinium methanesulfonate, triethylammonium (p-bromobenzene)sulfonate, tetraethylammonium (p-bromobenzene)sufonate, tetraethylammonium(p-toluene)sulfonate, pyridinium(p-toluene)sulfonate and pyridinium-3-nitrobenzenesulfonate; more preferred is triethylammonium methanesulfonate; and
finally (c), the conjugate anion of a sulfonic acid may be generated *in-situ* by reacting 2-deoxy-2,2-difluoro-D-ribofuranose with a sulfonic anhydride such as benzenesulfonic anhydride, p-bromobenzenesulfonic anhydride or methanesulfonic anhydride, in a base such as triethylamine. The products of the reaction are for example 2-deoxy-2,2-difluoro-D-ribofuranosyl-3,5-di-O-benzoyl-1-methanesulfonate and triethylammonium methanesulfonate.

The beta-anomer ribofuranosyl sulfonate and conjugate anion sulfonic acid are heated from about 50°C to about 130°C and more preferably to the reflux temperature of the solvent mixture.

Solvents suitable for use in the anomerization process must be inert to the reaction conditions; preferred are acetonitrile, 1,2-dichloroethane, 1,1,2-trichloroethane, chlorobenzene, bromobenzene, dichlorobromomethane, anisole, glyme, diglyme, methyl tert-butyl ether, tetrahydrofuran, dioxane, ethyl acetate, toluene, xylenes, pyridine, N-methylpyrrolidinone, N,N-dimethylformamide, 1,3-dimethyl-2-imidazolidinone, N,N-dimethylacetamide, and mixtures thereof; most preferred are anisole, toluene, glyme, acetonitrile, and mixtures thereof.

A catalyst selected from crown ethers or phase transfer catalyst may be added to increase the solubility and nucleophilicity of metal salts used as the source of the conjugate anion of the sulfonic acid; preferred catalyst are selected from 18-Crown-6, 15-Crown-5, 12-Crown-4 and tris[2-(2-methoxyethoxy)ethyl]amine.

This process is carried out under atmospheric conditions and preferably anhydrous and conditions is substantially complete in about 15 minutes to about 24 hours. The resulting alpha-anomer enriched carbohydrates of formula (II) are prepared in an anomeric ratio of from about 2.3:1 to 3.0:1 alpha to beta.

α anomer enriched intermediates of Formula (II) wherein T is hydrogen may be prepared in a similar manner, except a lactol of Formula (IV) is used as the starting material.

Glycosylation reactions typically require protecting the hydroxy groups of the lactol prior to their use to prevent their hydroxy groups from reacting with the nucleobase, or being decomposed in some manner. Hydroxy protecting groups (X), suitable for use in the present glycosylation process, are independently chosen from known protecting groups used in synthetic organic chemistry. Each hydroxy protecting group selected is preferably capable of being efficiently placed on the lactol and easily removed therefrom once the glycosylation reaction is completed. Hydroxy protecting groups known in the art are described in Chapter 3 of Protective Groups in Organic Chemistry, McOmie Ed., Plenum Press, New York (1973), and Chapter 2 of Protective Groups in Organic Synthesis, Green, John, J. Wiley and Sons, New York (1981); preferred are ester forming groups such as formyl, acetyl, substituted acetyl, propionyl, butynyl, pivaloyl, 2-chloroacetyl, benzoyl, substituted benzoyl, phenoxycarbonyl, methoxyacetyl; carbonate derivatives such as phenoxycarbonyl, ethoxycarbonyl, t-butoxycarbonyl, vinyloxycarbonyl, 2,2,2-trichloroethoxycarbonyl and benzyloxycarbonyl; alkyl ether forming groups such as benzyl, diphenylmethyl, triphenylmethyl, t-butyl, methoxymethyl, tetrahydropyranyl, allyl, tetrahydrothienyl, 2-methoxyethoxy methyl; and silyl ether forming groups such as trialkylsilyl, trimethylsilyl, isopropyldialkylsilyl, alkyldiisopropylsilyl, triisopropylsilyl, t-butyldialkylsilyl and 1,1,3,3-tetraisopropyldisiloxanyl; carbamates such as N-phenylcarbamate and N-imidazoyl- carbamate; however more preferred are benzoyl, mono-substituted benzoyl and disubstituted benzoyl, acetyl, pivaloyl, triphenylmethyl ethers, and silyl ether protecting groups, especially t-butyldimethylsilyl; while most preferred is benzoyl.

In attaching the hydroxy protecting groups to the lactol, typical reaction conditions are employed and depend on the nature of the hydroxy protecting group chosen. Typical reaction conditions are described in U.S. Patent 4,526,988.

In accordance with the present process, at least an equimolar amount of nucleobase (R") is employed, relative to the amount of carbohydrate employed. However, it is more preferable to use an excess of nucleobase ranging from about 1 molar equivalent to 30 molar equivalents; more preferably from about 10 molar equivalents to 20 molar equivalents; and most preferably from about 15 molar equivalents to about 20 molar equivalents.

The nucleobases (R") employed herein are commonly known to organic chemists and no discussion of their synthesis is necessary. However, in order to be useful in the present glycosylation process, the nucleobase or their tautomeric equivalents that bear amino or hydroxy groups should preferably contain protecting groups such as primary amino protecting groups (W) and/or hydroxy protecting groups (Z), depending on the nature of the nucleobase. The protecting group prevents the hydroxy or amino groups from providing a competing reaction site for the α -anomer enriched carbohydrate. The protecting groups are attached to the nucleobase (R") before it is reacted with the alpha-anomer enriched carbohydrate of formula II and are removed subsequent thereto. A procedure for attaching the protecting groups to the nucleobases is described in U. S. Patent 4,526,988.

Preferred amino protecting groups (W) for pyrimidine nucleobases are selected from the group consisting of silyl ether protecting groups such as trialkylsilyl, t-butyldialkylsilyl and t-butyldiarylsilyl; carbamates such as t-butoxycarbonyl, benzyloxycarbonyl, 4-methoxybenzyloxycarbonyl, and 4-nitrobenzyloxycarbonyl; formyl, acetyl, benzoyl and pivalamido; ether forming groups such as methoxymethyl, t-butyl, benzyl, allyl and tetrahydropyranyl; more preferred is trimethylsilyl. Preferred amino protecting groups (W) for purine nucleobases are selected from the group consisting of alkylcarboxamides, haloalkylcarboxamides and arylcarboxamides such as 2-trialkylsilylethoxymethyl, 4-methoxybenzyl, 3,4-dimethoxybenzyl, t-butyl, phthalamido, tetrahydropyranyl, tetrahydrofuranyl, methoxymethyl ether, methoxythiomethyl, trityl, pivalamido, t-butyldimethylsilyl, t-hexyldimethylsilyl, triisopropylsilyl, trichloroethoxycarbonyl, trifluoroacetyl, naphthoyl, formyl, acetyl; sulfonamides such as alkylsulfonamido and arylsulfonamido, and more preferred is pivalamido. Besides serving as an amino protecting group, the pivalamido protecting group increases the solubility of notoriously insoluble purine nucleobase derivatives and directs the N-glycosidic coupling of the purine bases to the 9 regioisomer as opposed to the 7 regioisomer.

Preferred hydroxy protecting groups (Z) for pyrimidine nucleobases are selected from silyl ether forming groups such as trialkylsilyl; carbamates such as t-butoxycarbonyl, benzyloxycarbonyl, 4-methoxybenzyloxycarbonyl and 4-nitrobenzyloxycarbonyl; carbocyclic esters such as formyl, acetyl, and pivalamido; preferred is trimethylsilyl. Preferred hydroxy protecting groups (Z) for purine nucleobases are selected from the group consisting of ether forming groups such as benzyl, t-butyl, trityl, tetrahydropyranyl, tetrahydrofuranyl, methoxymethyl; ester forming groups such as formyl, acetyl, propionyl, pivaloyl, benzoyl, substituted benzoyl; carbonates such as carbobenzoxy, t-butoxycarbonyl, carbethoxy, vinyloxycarbonyl; carbamates, such as N,N-dialkylcarbamoyl; trialkylsilyl ethers such as t-butyltrimethylsilyl, t-hexyldimethylsilyl, triisopropylsilyl; more preferred is pivaloyl.

In providing protecting groups to the nucleobases of the present process, the protecting group itself may be protected. For example, N-acetylcytosine may be protected with trimethylsilyl to give bis-trimethylsilyl-N-acetylcytosine.

In addition, it is often advisable to convert any keto oxygen atoms on the nucleobase to enol form. This makes the nucleobase more aromatic and enhances the reactivity of the nucleobase with the alpha-anomer enriched carbohydrate of formula (II). It is most convenient to enolize the keto oxygens and provide silyl protecting groups for them.

Although not critical, it is advisable that the reaction between the α anomer enriched carbohydrate of formula (II) and the nucleobase be carried out in a dry atmosphere, e.g. in dry air, nitrogen or argon. This is because certain nucleobase derivatives such as silylated nucleobase derivatives are moisture sensitive.

Any solvents used to prepare the nucleobase may be removed prior to the glycosylation reaction or admixed with the reaction solvent, provided the admixture is inert to the glycosylation reaction.

In the case where the glycosylation reaction is carried out in a reaction solvent, the solvent must be inert to the glycosylation reaction. However, as previously mentioned, the particular reaction solvent employed will depend on the glycosylation reaction conditions (e.g. reaction temperature, solvent), leaving group and nucleobase employed.

The glycosylation reaction can be carried out at a temperature ranging from about 170°C to about -120°C under atmospheric conditions and is typically substantially complete in about 5 minutes to about 20 hours.

The progress of the present process may be followed by procedures well known to one of ordinary skill in the art such as high pressure liquid chromatography (HPLC) or thin layer chromatography (TLC) which can be used to detect the formation of nucleoside product.

When the reaction is effected in solution, it is preferred that a high boiling inert solvent and a solution having a carbohydrate concentration of at least a 20 percent carbohydrates be used. A carbohydrate concentration of from about 20 percent to about 70 percent is preferred; and about 30 percent to about 70 percent is more preferred; while about 30 percent to about 50 percent is most preferred. Suitable reaction temperatures range from about 70°C to about 170°C.

The high boiling solvent preferably has a boiling point above about 70°C and is selected from the group consisting of non-nucleophilic, aromatic, haloalkyl, alkoxy and halo substituted aromatic solvents, and mixtures thereof. Preferred are solvents are 1,2-dichloroethane, 1,1,2-trichloroethane, glyme, diglyme, toluene, xylenes, anisole, dichlorobromomethane, chlorobenzene, dibromochloromethane, tribromomethane, dibromomethane, acetonitrile, propionitrile, dioxane, and mixtures thereof; while more preferred is anisole.

The α anomer enriched carbohydrate of formula (II) used with high boiling solvents contain a sulfonoyloxy group selected from alkylsulfonyloxy, arylsulfonyloxy, substituted alkylsulfonyloxy and substituted arylsulfonyloxy such as, methanesulfonyloxy, 2-chloro-1-ethanesulfonyloxy, toluenesulfonyloxy, p-nitrobenzenesulfonyloxy and p-bromobenzenesulfonyloxy.

The nucleobase (R") preferred for use with high boiling solvents are those selected from the group consisting of 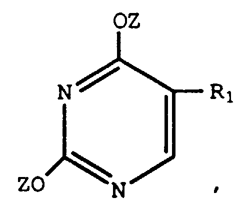 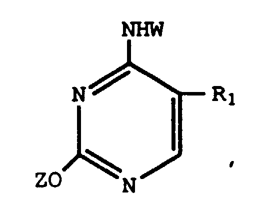 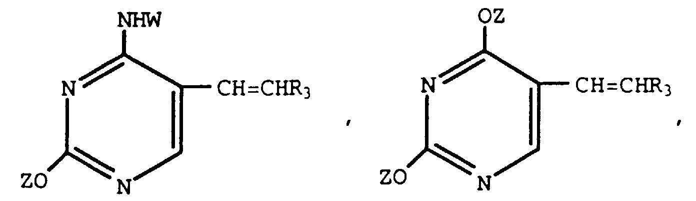 and 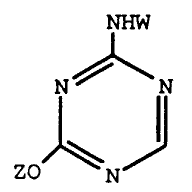 wherein R₁, R₃, Z and W are as defined above.

When the α anomer enriched carbohydrate of formula (II) contains a fluoro sulfonyloxy group it is unstable at temperatures above room temperature. Therefore, glycosylation reactions employing these sulfonyloxy groups must be carried out a temperature at or below room temperature. When the glycosylation reaction is run under these conditions the solvent must be low freezing. Preferred temperatures for the reaction range from about 25°C to about -120°C. In which case, the preferred solvents are selected from the group consisting of dichloromethane, 1,2-dichloroethane, dichlorofluoromethane, acetone, toluene, anisole, chlorobenzene, and mixtures thereof; more preferred is dichloromethane. However, the optimum glycosylation reaction temperature employed at low temperatures depend on the leaving group (Y). For example, when the leaving group is trifluoromethanesulfonyloxy, the preferred reaction temperature ranges from about -50°C to about 25°C; while about -20°C to about 25°C is more preferred. However, when the leaving group is 1,1,1-trifluoroethanesulfonyloxy, octafluorobutanesulfonyloxy or nonafluorobutanesulfonyloxy, the preferred reaction temperature ranges from about -20°C to about 25°C; while about 0°C to about 25°C is more preferred.

The nucleobase (R") preferred for use under low temperature conditions are those selected from the group consisting of 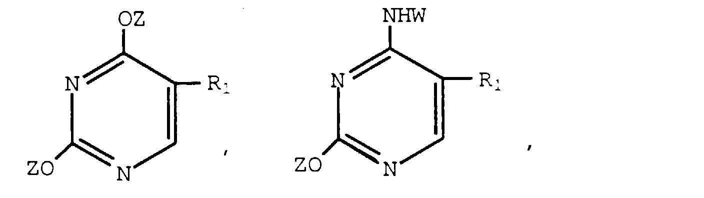 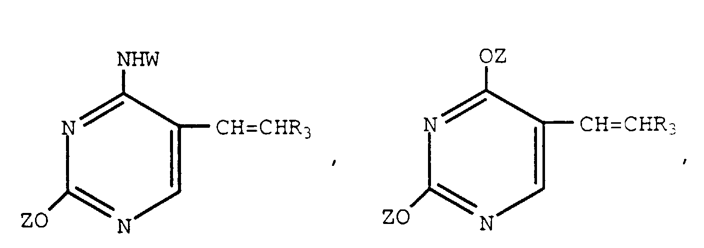 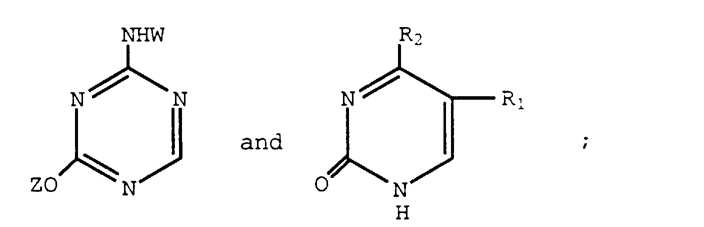 wherein R₁, R₂, R₃, Z and W are as defined above.

The nucleobase (R'') may optionally be converted to a metal cation salt to enhance its nucleophilic reactivity with the alpha-anomer enriched carbohydrate of formula (II) (i.e. anion glycosylation). These nucleobase cation salts are prepared by adding a base to the nucleobase in a solvent. The base may be selected from the group consisting of sodium t-butoxide, sodium hydride, sodium methoxide, sodium ethoxide, lithium hydride, potassium hydride, potassium hydroxide, potassium methoxide, potassium ethoxide and potassium t-butoxide. Alternatively, the base may be selected from trialkylamine or tetraalkylammonium. Suitable inert solvents for the reaction may be selected from the group consisting of acetonitrile, dimethylformamide, dimethylacetamide, 1,3-dimethyl-2-imidazolidinone, tetrahydrofuran, sulfolane, N-methylpyrrolidinone, dimethylsulfoxide, and mixtures thereof. The solvent may be removed prior to the glycosylation reaction or admixed with the glycosylation reaction solvent, provided the admixture is substantially inert to the glycosylation reaction. Suitable reaction temperatures range from about 23°C to about 130°C

The nucleobase (R") is preferably selected from the group consisting of 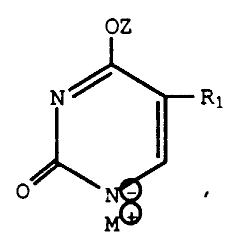 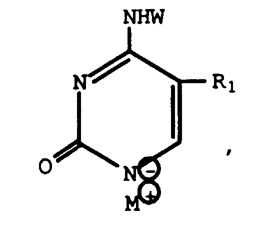 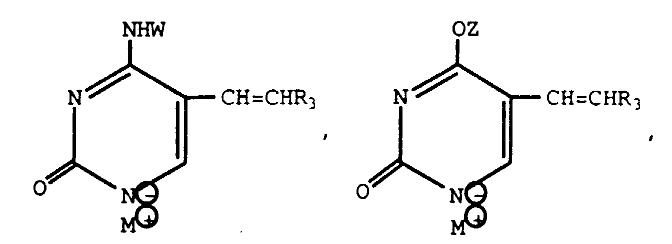 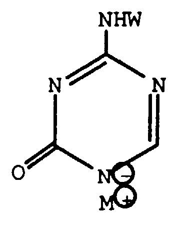 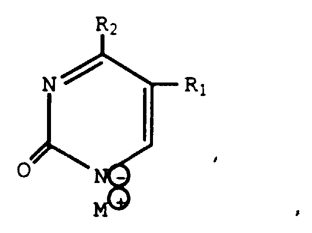 and 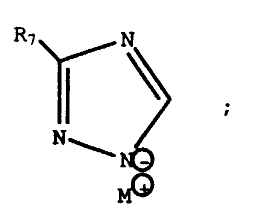 wherein R₁ through R₇, Z, W and M⁺ are as defined above.

The α anomer enriched carbohydrate of formula (II) under these conditions contains a sulfonoyloxy group selected from alkylsulfonyloxy, arylsulfonyloxy, substituted alkylsulfonyloxy, substituted arylsulfonyloxy, fluoro alkylsulfonyloxy and fluoro arylsulfonyloxy such as, trifluoromethanesulfonyloxy, 1,1,1-trifluoroethanesulfonyloxy, octafluorobutanesulfonyloxy, nonafluorobutane -sulfonyloxy, methanesulfonyloxy, 2-chloro-1-ethanesulfonyloxy, toluenesulfonyloxy, p-nitrobenzenesulfonyloxy and p-bromobenzenesulfonyloxy.

As previously noted, fluoro sulfonyloxy groups of formula (II) tend to be unstable at higher temperatures and the above reaction with the metal cation salt nucleobases should be effected using a low freezing inert solvent with such groups. Preferred temperatures for the reaction range from about 25°C to about -120°C.

The glycosylation reaction may also be run in the absence of solvent (i.e. fusion glycosylation). Obviously, the temperature employed must be sufficient to convert the α anomer enriched carbohydrate intermediate of formula (II) and nucleobase to molten phase. Preferred reaction temperatures range from about 100°C to about 160°C; however more preferred is about 110°C to about 150°C; while most preferred is about 130°C to about 150°C.

The α anomer enriched carbohydrate of formula (II) under fusion conditions contain a sulfonoyloxy group selected from alkylsulfonyloxy, arylsulfonyloxy, substituted alkylsulfonyloxy and substituted arylsulfonyloxy such as, methanesulfonyloxy, 2-chloro-1-ethanesulfonyloxy, toluenesulfonyloxy, p-nitrobenzenesulfonyloxy and p-bromobenzenesulfonyloxy.

The nucleobase (R") suitable for use under fusion conditions is preferably selected from the group consisting of 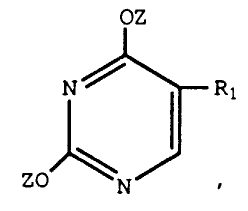 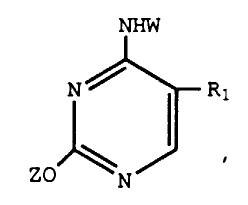 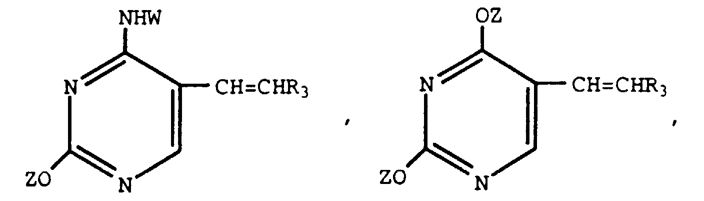 and 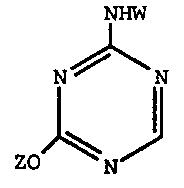 wherein wherein R₁, R₃, Z and W are as defined above.

The present process may also be promoted by a catalyst. When a catalyst is employed, it substantially reduces the amount of nucleobase required, increases stereoselectivity, lowers processing cost, increases processing through-put, simplifies the product separation, and lowers the necessary reaction temperature allowing for the use of less thermally stable carbohydrates. Therefore, the present process desirably employs a catalyst which is a salt containing a non-nucleophilic anion. Preferred are Group IA, Group IIA or quaternary ammonium salts. The catalyst should be soluble in the reaction solvent and highly ionized. Preferred are salt catalysts selected from the group consisting of potassium, barium, cesium and trialkylammonium salts of trifluoromethanesulfonic acid, nanofluorobutane sulfonic acid, sulfuric acid, perchloric acid, nitric acid, and trifluoroacetic acid; more preferred are potassium or cesium salts of trifluoromethanesulfonic acid. Suitable reaction temperatures range from about 50°C to about 100°C.

The solvent is preferably selected from polar, non-nucleophilic solvents such as glyme, diglyme, anisole, acetonitrile, propionitrile, dioxane, and mixtures thereof; while more preferred is acetonitrile.

The α anomer enriched carbohydrate of formula (II) under catalytic conditions preferably contain a sulfonyloxy group selected from alkyl sulfonyloxy and aryl sulfonyloxy groups such as, methanesulfonyloxy, 2-chloro-1-ethanesulfonyloxy, toluenesulfonyloxy, p-nitrobenzenesulfonyloxy and p-bromobenzenesulfonyloxy.

The nucleobase (R") for use under catalytic conditions is preferably selected from the group consisting of 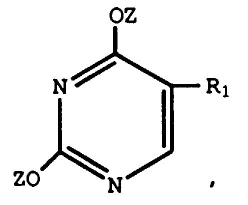 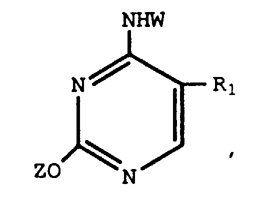 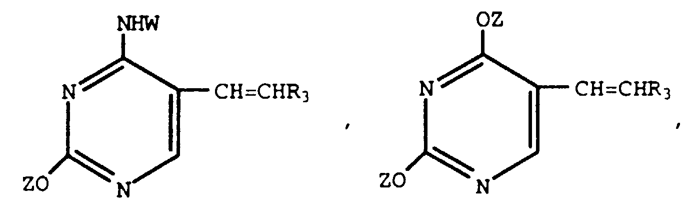 and 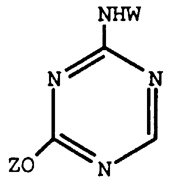 wherein R₁, R₂, R₃, Z and W are as defined above.

The final phase of the reaction sequence is the removal of the protecting groups X, Z and/or W (i.e. deblocking) from the blocked nucleoside of formula (I). The same anomeric ratio of nucleosides is obtained upon removal of the protecting groups.

Most silyl and silyl-amino protecting groups are easily cleaved by use of a protic solvent, such as water or an alcohol. The acyl protecting groups, such as benzoyl and the acyl-amino protecting groups, are removed by hydrolysis with a strong base at a temperature from about 0°C to about 100°C. Strong or moderately strong bases suitable for use in this reaction are bases which have a pKa (at 25°C) of about 8.5 to about 20.0. Such bases include alkali metal hydroxides such as sodium or potassium hydroxide; alkali metal alkoxides such as sodium methoxide or potassium t-butoxide; alkali metal amides; amines such as diethylamine, hydroxylamine, ammonia and the like; and other common bases such as hydrazine and the like. At least one equivalent of base is needed for each protecting group.

The acyl protecting groups can also be removed with acid catalysts, such as methanesulfonic acid, hydrochloric acid, hydrobromic acid, sulfuric acid, or with acidic ion exchange resins. It is preferrable to carry out such hydrolysis at relatively high temperatures, such as the reflux temperature of the mixture, but temperatures as low as ambient may be used when particularly strong acids are employed.

The removal of ether protecting groups is carried out by known methods, for example, with ethanethiol and aluminum chloride.

The t-butyldimethylsilyl protecting group requires acid conditions, such as contact with gaseous hydrogen halide, for its removal.

Removal of the protecting groups may be conveniently carried out in alcoholic solvents, especially aqueous alkanols such as methanol. However, the deblocking reaction may also be carried out in any convenient solvent, such as polyols including ethylene glycol, ethers such as tetrahydrofuran, ketones such as acetone and methyl ethyl ketone, or dimethylsulfoxide.

In a preferred embodiment, the deblocking reaction employs ammonia to remove a benzoyl hydroxy-protecting group at a temperature of about 10°C. It is preferable, however, to use an excess of base in this reaction, although the amount of excess base used is not crucial.

In accordance with the present process, βanomer enriched nucleosides are prepared in an α to β anomer ratio greater than 1:1 to about 1:9.

The resulting β anomer enriched nucleoside of formula (I) may be extracted and/or isolated from the reaction mixture as described in U.S. Patent 4,965,374, which is incorporated herein by reference.

The following examples illustrate specific aspects of the present invention and are not intended to limit the scope thereof in any respect and should not be so construed.

### Example 1

### Preparation of beta-anomer enriched 1-(2'-deoxy-2',2'-difluoro-3',5'-di-O-benzoyl-D-ribofuranosyl)-4-aminopyrimidin-2-one with 10 equivalents of bis-trimethylsilylcytosine

Bis-trimethylsilylcytosine was prepared by combining 2.44 g of cytosine, 5.15 ml of hexamethyldisilazane and 580 mg of ammonium sulfate with 5 ml of xylenes and refluxing the solution at 120°C for 1 hour. An additional 5 ml of hexamethyldisilazane were added to form a homogenous solution which was refluxed for 30 minutes. The xylenes and excess hexamethyldisilazane were removed and a gelatin-like bis-trimethylsilylcytosine formed. 5.6 g of the bis-trimethylsilylcytosine were reconstituted in 20 ml of xylenes. The xylenes were removed and the bis-trimethylsilylcytosine was again reconstituted in 20 ml of xylenes. The bistrimethylsilylcytosine was evaporated to dryness and reconstituted in 5 ml of xylenes. 1 g of 2-deoxy-2,2-difluoro-D-ribofuranosyl-3,5-dibenzoyl-1-α-methanesulfonate was reacted with the bis-trimethylsilylcytosine solution at 127°C for 3.5 hours. HPLC analysis confirmed completion of the reaction.

To extract the nucleoside product, the reaction mixture was cooled to 60°C, diluted in 100 ml of ethyl acetate and washed with 200 ml of 1 N hydrochloric acid. An emulsion occurred and the two layers that formed were separated. The organic layer was washed successively with 100 ml of 5% sodium bicarbonate, and 100 ml of saturated sodium chloride solution then dried over magnesium sulfate. A quantitative HPLC analysis of the ethyl acetate layer indicated that the yield of blocked beta-anomer nucleoside was 50 per cent. The beta to alpha anomeric ratio of the blocked nucleoside was 2.2:1.

### Example 2

### Preparation of beta-anomer enriched 1-(2'-deoxy-2',2'-difluoro-3',5'-di-O-benzoyl-D-ribofuranosyl)-4-aminopyrimidin-2-one with 5 equivalents of bis-trimethylsilylcytosine

To 2.8 g of bis-trimethylsilylcytosine were added 3 ml of xylenes and the solution heated to 120°C until the bis-trimethylsilylcytosine solubilized. 1 g of 2-deoxy-2,2-difluoro-D-ribofuranosyl-3,5-dibenzoyl-1-α -methanesulfonate, dissolved in 2 ml of xylenes, was heated and reacted with the bis-trimethylsilylcytosine solution at 130°C for 16 hours. HPLC analysis confirmed completion of the reaction. The beta to alpha anomeric ratio of the blocked nucleoside was 1.1:1.

To extract the nucleoside product, the reaction mixture was diluted with 150 ml ethyl acetate and washed with 150 ml 1 N hydrochloric acid. An emulsion occurred and the two layers that formed were separated. The organic layer was washed successively with 100 ml of water, and 100 ml of 5% sodium bicarbonate then dried over magnesium sulfate. For a more accurate HPLC analysis, 1 ml of the organic layer was evaporated to dryness and reconstituted in 1 ml of the acetonitrile. A quantitative HPLC analysis of the organic layer in acetonitrile indicated that the yield of blocked beta-anomer nucleoside was 36 percent.

### Example 3

### Preparation of beta-anomer enriched 1-(2'-deoxy-2',2'-difluoro-3',5'-di-O-benzoyl-D-ribofuranosyl)-4-aminopyrimidin-2-one hydrochloride salt with 15 equivalents of bis-trimethylsilylcytosine

Bis-trimethylsilylcytosine was prepared by combining 18.33 g of cytosine and 10 ml of anisole with 64.3 ml of N-methyl-N-(trimethylsilyl)-trifluoroacetamide and heating the solution at 80°C for 30 minutes. 5.0 g of 2-deoxy-2,2-difluoro-D-ribofuranosyl-3,5-dibenzoyl-1-α -methanesulfonate, dissolved in 10 ml anisole, were reacted with the bis-trimethylsilylcytosine solution at 105°C for 5 hours. HPLC analysis confirmed completion of the reaction. The beta to alpha anomeric ratio of blocked nucleoside was 5.4:1.

To isolate the nucleoside product, the reaction mixture was cooled to 60°C, diluted with 75 ml of ethyl acetate and washed with 200 ml of 1 N hydrochloric acid. A semi-clear solution containing solid particulates formed. The solution was warmed to 60°C-70°C for 15 minutes, filtered, and the isolated solid was washed successively with 20 ml of ethyl acetate then dried in a vacuum oven at 40°C for 16 hours. The resulting nucleoside product weighed 4.0 g, m.p. 252°C-256°C. A quantitative HPLC analysis confirmed that the product was the hydrochloride salt of the blocked beta-anomer nucleoside in a yield of 75 percent.

### Example 4

### Preparation of beta-anomer enriched 1-(2'-deoxy-2',2'-difluoro-3',5'-di-O-benzoyl-D-ribofuranosyl)-4-aminopyrimidin-2-one with 10 equivalents of bis-trimethylsilylcytosine

Bis-trimethylsilylcytosine was prepared by the procedure described in Example 1, except 20 g of cytosine, 380 ml of hexamethyldisilazane, 1.18 g of ammonium sulfate and 48 ml of xylenes were used. The bis-trimethylsilylcytosine was reconstituted in 24 ml xylenes. 9.6 g of 2-deoxy-2,2-difluoro-D-ribofuranosyl-3,5-dibenzoyl-1-toluenesulfonate, in a 70:30 alpha to beta ratio, were dissolved in 24 ml of xylenes and reacted with the bis-trimethylsilylcytosine solution for 1 hour. HPLC analysis confirmed completion of the reaction.

To extract the nucleoside product, the reaction mixture was cooled to 65°C and 100 ml of ethyl acetate were added. The solution was maintained at 65°C and washed with 200 ml of 1N hydrochloric acid. An emulsion occurred and the two layers that formed were separated. The organic layer washed with 200 ml of 5% sodium bicarbonate then dried over magnesium sulfate. The beta to alpha anomeric ratio of the blocked nucleoside was 1.1:1. A quantitative HPLC analysis indicated that the yield of blocked beta-anomer nucleoside was 27 percent.

### Example 5

### Preparation of beta-anomer enriched 1-(2'-deory-2',2'-difluoro-3',5'-di-O-benzoyl-D-ribofuranosyl)-4-aminopyrimidin-2-one with 20 equivalents of bis-trimethylsilylcytosine

Bis-trimethylsilylcytosine was prepared by combining 30 g of cytosine with 175 ml of hexamethyldisilazane and 25 mg of ammonium sulfate under nitrogen and heating the solution at 120°C for 2 hours. The mixture was cooled to 80°C and diluted with 100 ml of ethyl acetate. The hexamethyldisilazane and ethyl acetate were subsequently atmospherically distilled at a temperature of 145°C. This procedure was repeated twice then the resulting bis-trimethylsilylcytosine was added to 15 ml of anisole and cooled to 110°C-115°C. 5.75 g of 2-deoxy-2,2-difluoro-D-ribofuranosyl-3,5-dibenzoyl-1-α -methanesulfonate, dissolved in 10 ml of anisole, were stirred at 45°C until a homogeneous solution formed and reacted with the bis-trimethylsilylcytosine solution at 115°C-120°C for 7 hours. HPLC analysis confirmed completion of the reaction. The beta to alpha anomeric ratio of blocked nucleoside was 7.3:1.

To isolate the nucleoside product, the reaction mixture was cooled to 88°C, diluted with 34 ml of ethyl acetate and washed with 125 ml of 4 N hydrochloric acid. A slurry containing solid particulates formed and was stirred for 1 and 1/2 hours at 80°C and filtered. The filtrate was washed with 50 ml of 4 N hydrochloric acid and dried in a vacuum oven at 45°C. The resulting nucleoside product weighed 4.6 g. A quantitative HPLC analysis indicated that the yield of blocked beta-anomer nucleoside was 79.5 percent.

### Example 6

### Preparation of beta-anomer enriched 1-(2'-deoxy-2',2'-difluoro-3',5'-di-O-benzoyl-D-ribofuranosyl)-4-aminopyrimidin-2-one hydrochloride salt with 20 equivalents of bis-trimethylsilylcytosine

Bis-trimethylsilylcytosine was prepared by the procedure described in Example 5. The solution was cooled to 100°C. 5.75 g of 2-deoxy-2,2-difluoro-D-ribofuranose-3,5-dibenzoyl-1-α-methanesulfonate, dissolved in 10 ml of anisole, were stirred at 45°C until a homogeneous solution formed and reacted with the bis-trimethylsilylcytosine solution at 110°C-115°C for 16 hours. HPLC analysis confirmed that only 3.9 percent of unreacted 2-deoxy-2,2-difluoro-D-ribofuranosyl-3,5-dibenzoate-1-α-methanesulfonate remained. The beta to alpha anomeric ratio of blocked nucleoside was 7.2:1.

To extract the nucleoside product, the reaction mixture was cooled and diluted with 69 ml of ethyl acetate at 65°C. The reaction mixture was then combined with 185 ml of 4 N hydrochloric acid. The mixture was refluxed for 1 hour at 78°C to form a slurry. The slurry was filtered and the solid was washed with 60 ml of 4 N hydrochloric acid and dried in a vacuum oven at 45°C. The nucleoside product weighed 3.62 g. A quantitative HPLC analysis confirmed that the product was the hydrochloride salt of the blocked beta-anomer nucleoside in a yield of 64.2 percent.

### Example 7

### Preparation of beta-anomer enriched 9-(2'-deoxy-2',2'-difluoro-3',5'-di-O-benzoyl-D-ribofuranosyl)-5-aminopurine with 15 equivalents of bis-trimethylsilyladenine

Bis-trimethylsilyladenine was prepared by combining 7 g of adenine and 109 ml of hexamethyldisilazane with 250 mg of ammonium sulfate and heating the mixture at 110-115°C for 8 hours. The solution was refluxed for an additional 30 minutes and the excess hexamethyldisilazane subsequently removed and 14.5 g of the bis-trimethylsilyladenine were reconstituted in 3 ml of anisole. 1.58 g of 2-deoxy-2,2-difluoro-D-ribofuranosyl-3,5-dibenzoyl-1-α-methanesulfonate were reacted with the bis-trimethylsilyladenine solution at 105°C-110°C for 24 hours. HPLC analysis confirmed completion of the reaction.

To extract the nucleoside product, the reaction mixture was cooled to 30°C, diluted with 50 ml of ethyl acetate and washed with 75 ml of 4 N hydrochloric acid. An emulsion occurred and the organic layer was separated and washed successively with 75 ml of 5% sodium bicarbonate, and 75 ml of saturated sodium chloride solution then dried over magnesium sulfate. The beta to alpha anomeric ratio of the blocked nucleoside was 6:1.

The following Table shows how the carbohydrate concentration and carbohydrate selected effects the anomeric ratio of the nucleoside product.

**Table**

| Solvent | Carbo. | Base (R') | Base (R') Equiv. | Temp. | Carbo Conc. | α/β Nucleoside Ratio | Yield |
|---|---|---|---|---|---|---|---|
| Xylenes | α-OMs | Cytosine | 1.5 | 127°C | 20% | 1.5:1 | 14% β |
| Xylenes | α-OMs | Cytosine | 1.5 | 127°C | 50% | 1.5:1 | 15% β |
| Xylenes | α-OMs | Cytosine | 5 | 130°C | 20% | 1:1.1 | 36% β |
| Xylenes | α-OMs | Cytosine | 10 | 127°C | 50% | 1:2.2 | 50% β |
| Xylenes | α-OMs | Cytosine | 10 | 120°C | 20% | 1:1.6 | 32% β |
| Xylenes | 50:50 α/β-OMs | Cytosine | 1.5 | 125°C | 50% | 3:1 | 12% β |
| Anisole | α-OMs | Cytosine | 2 | 105°C | 20% | 1.3:1 | 18% β |
| Anisole | α-OMs | Cytosine | 3 | 105°C | 50% | 1:1.3 | 22% β |
| Anisole | α-OMs | Cytosine | 15 | 105°C | 50% | 1:5.4 | 75% β(a) |
| Anisole | α-OMs | 5-F-Cytosine | 10 | 115°C | 50% | 1:6 | N/D |
| Anisole | α-OMs | 5-F-Uracil | 5 | 130°C | 50% | 1:6 | N/D |
| Xylenes | 70:30 α:β-OTs | Cytosine | 3 | 123°C | 20% | 1.7:1 | 6% β |
| Xylenes | 70:30 α:β-OTs | Cytosine | 5 | 125°C | 20% | 1.7:1 | N/D |
| Xylenes | 70:30 α:β-OTs | Cytosine | 10 | 125°C | 20% | 1:1.1 | 27% β |
| Xylenes | 70:30 α:β-OTs | Cytosine | 10 | 125°C | 20% | 1.3:1 | 23% β |
| Xylenes | 85:15 α:β-OBs | N-Acetyl-Cytosine | 5 | 110°C | 20% | 1:1 | N/D |
| Anisole | α-OMs | Cytosine | 20 | 115°C | 25% | 1:7.3 | 79.5% β(a) |
| Anisole | α-OMs | Adenine | 15 | 110°C | 50% | 1:6 | N/D |
| Anisole | α-OMs | Cytosine | 20 | 115°C | 25% | 1:7.2 | 64% β |

(N/D) means not determined. The carbohydrates (Carbo.) are hydroxy protected and include α- or β-OMs is alpha- or beta-2,2-difluoro-2-deoxy-D-ribofuranosyl-3,5-dibenzoyl-1-methanesulfonate; β- or α-OTs is beta- or alpha-2,2-difluoro-2-deoxy-D-ribofuranosyl-3,5-dibenzoyl-1-toluenesulfonate; and α- or β-OBs is alpha-or beta-2,2-difluoro-2-deoxy-D-ribofuranosyl-3,5-dibenzoyl-1-bromo-benzenesulfonate. The 70:30 α:β-OTs carbohydrates were obtained by anomerizing β-OTs with a salt of p-toluenesulfonic acid. The yields are based on the amount of carbohydrate and were calculated from a quantitative reverse phase HPLC analysis, wherein the corresponding solution product peak was compared with a standard, 1-(2'-deoxy-2',2'-difluoro-3',5'-di-O-benzoyl-β-D-ribofuranosyl)-4-aminopyrimidin-2-one, except in (a) which is an isolated product yield. (*) The carbohydrate concentration (Carbo. Conc.) has units of percent carbohydrate by weight (grams) per unit volume of solvent (milliliters). The nucleobase protecting group in each example is trimethylsilyl.

### Example 8

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 112 ml of hexamethyldisilazane and 100 mg of ammonium sulfate. The solution was heated to 115°C-120°C for 1 and 1/2 hours with stirring and the excess hexamethyldisilazane subsequently removed. The mixture was cooled to 60°C and reconstituted in 40 ml of 1,2-dichloroethane to form a homogenous solution of bis-trimethylsilylcytosine.

To 1 g of 2-deoxy-2,2-difluoro-D-ribofuranosyl-3,5-dibenzoate was added 10 ml of dichloromethane and 0.54 ml of triethylamine. This solution was stirred at 23°C for 30 minutes, cooled to -78°C and reacted with 0.57 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-1-trifluoromethanesulfonate intermediate in solution. Care was taken to maintain the temperature of the reaction mixture below -65°C. An ¹⁹F nuclear magnetic resonance (NMR) analysis of the alpha-anomer enriched 2-deoxy-2,2-difluoro-D-ribofuranosyl-3,5-dibenzoyl-1-trifluoromethanesulfonate intermediate at 65°C provided the following data:
¹⁹F NMR (300 MHz, CDCl₃), δ-77 (s, 3F, CF₃SO₂-), -111 (d, J=257 Hz, 1F, alpha-anomer), -122 (d, J=242 Hz, 1F, beta-anomer), -124 (d, J=257 Hz, 1F, alpha-anomer), -126 ppm (d, J=242 Hz, 1F, beta-anomer). It should be noted that all ¹⁹F NMR peak shifts are relative to hexafluorobenzene, which was assigned a frequency of -162.9 ppm. The ¹⁹F NMR spectrum also indicated fluorine - proton couplings however, the nature of these couplings were not determined.

The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at -65°C and the reaction temperature was allowed to rise to 23°C to form the titled blocked nucleoside which was confirmed by HPLC analysis. The beta to alpha anomeric ratio of the blocked nucleoside was 1.9:1.

To extract the nucleoside product from the reaction mixture, 100 ml of dichloromethane and 200 ml of 1 N hydrochloric acid were added. The organic layer was separated and washed with 200 ml of 5% sodium bicarbonate. The organic layer was again separated and washed with 200 ml of saturated sodium chloride. The titled nucleoside product precipitated from the organic layer A quantitative HPLC analysis revealed a yield of blocked beta-anomer nucleoside of 42 percent. ¹H NMR (DMSO): δ = 4.74(4'H), 4.79 (5'H), 5.84 (5H), 5.88 (3'H), 6.44 (1'H), 7.56 (NH₂), 7.68 (6H). ¹³C NMR (DMSO): δ= 63.46 (5'C), 71.80 (3'C), 75.71 (4'C), 84.64 (1'C), 95.12 (5C), 121.86 (2'C), 141.93 (6C), 154.48 (2C), 165.87 (4C).

### Example 9

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

A bis-trimethylsilylcytosine solution was prepared by suspending 5.78 g of cytosine in 75 ml of dichloromethane and adding 20.57 ml of N-methyl-N-trimethylsilyltrifluoroacetamide and cooling the resulting solution to -30°C.

To 1 g of 2-deoxy-2,2-difluoro-D-ribofuranosyl-3,5-dibenzoate was added 10 ml of dichloromethane and 0.55 ml of triethylamine. This solution was stirred at 23°C for 30 minutes, cooled to -78°C and reacted with 0.57 ml of trifluoromethanesulfonyl anhydride in 1 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranose-3,5-dibenzoyl-1-trifluoromethanesulfonate solution was reacted with the bis-trimethylsilylcytosine solution at -30°C to form the titled blocked nucleoside which was confirmed by HPLC analysis. The beta to alpha anomeric ratio of the blocked nucleoside was 2.3:1.

To extract the nucleoside product from the reaction mixture, 200 ml of 1 N hydrochloric acid were added. The organic layer was separated and washed with 5% sodium carbonate. A quantitative HPLC analysis of the organic layer revealed a yield of blocked beta-anomer nucleoside of 45 percent.

### Example 10

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

A bis-trimethylsilyl cytosine solution was prepared by the procedure described in Example 8 and cooled to -15°C.

To 1 g of 2-deoxy-2,2-difluoro-D-ribofuranosyl-3,5-dibenzoate was added 10 ml of dichloromethane and 0.54 ml of triethylamine. This solution was stirred at 23°C for 30 minutes, cooled to -78°C and reacted with 0.57 ml of trifluoromethanesulfonyl anhydride in 0.5 ml of dichloromethane to form an alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at -15°C to form the titled blocked nucleoside which was confirmed by HPLC analysis. The beta to alpha anomeric ratio of the blocked nucleoside was 2.3:1.

To extract the nucleoside product from the reaction mixture, the dichloromethane was removed and the resulting residue was reconstituted in 21 ml of anisole and 40 ml of water then heated to 90°C. The solids that formed were removed from the solution. The organic and aqueous layers were separated and the organic layer was subsequently washed with an additional 10 ml of water. The beta-anomer nucleoside product precipitated from the organic layer. A quantitative HPLC analysis revealed a yield of blocked beta-anomer nucleoside of 58 percent.

### Example 11

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

A bis-trimethylsilylcytosine solution was prepared by suspending 5.78 g of cytosine in 20 ml of dichloromethane and adding 20.57 ml of N-methyl-N-trimethylsilyltrifluoroacetamide in 10 ml of dichloromethane and cooling the resulting solution to 0°C.

To 1 g of 2-deoxy-2,2-difluoro-D-ribofuranosyl-3,5-dibenzoate was added 10 ml of dichloromethane and 0.55 ml of triethylamine. This solution was stirred at 23°C for 30 minutes, cooled to -78°C and reacted with 0.57 ml of trifluoromethanesulfonyl anhydride in 1 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 0°C to form the titled blocked nucleoside which was confirmed by HPLC. The beta to alpha anomeric ratio of the blocked nucleoside was 2.5:1.

To extract the nucleoside product from the reaction mixture, 250 ml of 1 N hydrochloric acid were added. The organic layer was separated and washed with 200 ml of 5% sodium carbonate. A quantitative HPLC analysis of organic layer revealed a yield of blocked beta-anomer nucleoside of 49 percent.

### Example 12

### Preparation of beta-anomer enriched 1-(2'-deoxy-2',2'-difluoro-3',5'-di-O-benzoyl-D-ribofuranosyl)-4-acetamidopyrimidin-2-one with 10 equivalents of bis-trimethylsilyl-N-acetylcytosine

To 4 g of N-acetylcytosine were added 56 ml of hexamethyldisilazane and 698 mg of ammonium sulfate. This solution was heated to 115°C-120°C for 4 hours with stirring and the excess hexamethyldisilazane was subsequently removed. The mixture was cooled to 50°C and constituted in 50 ml of 1,2-dichloroethane. The 1,2-dichloroethane was removed and the resulting solid residue was reconstituted in 50 ml of 1,2-dichloroethane. The 1,2-dichloroethane was again removed and an oily residue formed. The oily residue was constituted in 2.5 ml 1,2-dichloroethane to form a homogenous bis-trimethylsilyl-N-acetylcytosine solution.

To 1 g of 2-deoxy-2,2-difluoro-D-ribofuranosyl-3,5-dibenzoate was added 2 ml of dry dichloromethane. This solution was cooled to -78°C and reacted with 0.55 ml of triethylamine and 0.58 ml of trifluoromethanesulfonyl anhydride to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilyl-N-acetylcytosine solution at 23°C. The reaction mixture was stirred at -60°C for 1 and 1/2 hour to form the titled blocked nucleoside which was confirmed by HPLC analysis. The beta to alpha anomeric ratio of the blocked nucleoside was 2:1.

To extract the nucleoside product from the reaction mixture, 50 ml of dichloromethane were added. The organic layer was separated and washed successively with 50 ml of 5% sodium bicarbonate then 50 ml of 1 N hydrochloric acid and 50 ml of saturated sodium chloride. A quantitative HPLC analysis of the organic layer revealed a yield of blocked beta-anomer nucleoside of 15 percent.

### Example 13

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 112 ml of hexamethyldisilazane and 50 mg of ammonium sulfate. The mixture was heated to 115°C-120°C for 3 hours with stirring and the excess hexamethyldisilazane was subsequently removed. This solution was then cooled to 27°C and a solid residue formed which was reconstituted in 35 ml of dichloromethane to form a homogenous bistrimethylsilylcytosine solution.

To 1 g of 2-deoxy-2,2-difluoro-D-ribofuranosyl-3,5-dibenzoate were added 10 ml of dichloromethane and 0.54 ml of triethylamine. The solution was cooled to -78°C and reacted with 0.57 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoate-1-trifluoromethanesulfonate solution was reacted with the bis-trimethylsilylcytosine solution at 27°C to form the titled blocked nucleoside which was confirmed by HPLC analysis and indicated that 11 percent of the alpha-anomer enriched 2-deoxy-2,2-difluoro-D-ribofuranosyl-3,5-dibenzoyl-1-trifluoromethanesulfonate remained unreacted. The beta to alpha anomeric ratio of the blocked nucleoside was 2.2:1. A quantitative HPLC analysis revealed a yield of blocked beta-anomer nucleoside of 54 percent.

### Example 14

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 112 ml of hexamethyldisilazane and 50 mg of ammonium sulfate. This solution was heated to 115°C-120°C for 2 hours with stirring and the excess hexamethyldisilazane was subsequently removed. The resulting oil was cooled to 23°C to form a solid residue which was reconstituted in 35 ml of dichloromethane to form a homogenous bistrimethylsilylcytosine solution and cooled to 0°C.

To 1 g of 2-deoxy-2,2-difluoro-D-ribofuranosyl-3,5-dibenzoate was added 9 ml of dichloromethane and 0.54 ml of triethylamine. The solution was cooled to -78°C and reacted with 0.57 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 23°C to form the titled blocked nucleoside which was confirmed by HPLC analysis. The beta to alpha anomeric ratio of the blocked nucleoside was 2.2:1.

To extract the nucleoside product from the reaction mixture was washed twice with 150 ml of 1 N hydrochloric acid. The organic layer was separated, washed with 150 ml of 5% sodium bicarbonate and washed again with 150 ml saturated sodium chloride. A quantitative HPLC analysis of the organic layer revealed a yield of blocked beta-anomer nucleoside of 49 percent.

### Example 15

### Preparation of beta-anomer enriched 1-(2'-deoxy-2',2'-difluoro-3',5'-di-O-benzoyl-D-ribofuranosyl)-4-aminopyrimidin-2-one with 30 equivalents of bis-trimethylsilylcytosine

To 5.9 g of cytosine were added 112 ml of hexamethyldisilazane and 25 mg of ammonium sulfate. The solution was heated to 120°C-125°C for 3 hours with stirring and the excess hexamethyldisilazane was subsequently removed. The resulting solid residue was reconstituted in 35 ml of dichloromethane and cooled to 10°C to form a homogenous bis-trimethylsilylcytosine solution.

To 655 mg of 2-deoxy-2,2-difluoro-D-ribofuranosyl-3,5-dibenzoate were added 0.55 ml of dichloromethane and 0.36 ml of triethylamine. The solution was stirred at 23°C for 30 minutes, cooled to -78°C and reacted with 0.35 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-l-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 10°C to form the titled blocked nucleoside which was confirmed by HPLC analysis. The beta to alpha anomeric ratio of the blocked nucleoside was 2.7:1. A quantitative HPLC analysis revealed a yield of blocked beta-anomer nucleoside of 60 percent.

### Example 16

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 112 ml of hexamethyldisilazane and 50 mg of ammonium sulfate. The solution was heated to 115°C-120°C for 1 and 1/2 hours with stirring and the excess hexamethyldisilazane was subsequently removed. The resulting solid residue was reconstituted in 40 ml of 1,2-dichloromethane at 23°C to form a homogenous bis-trimethylsilyl cytosine solution.

To 1 g of 2-deoxy-2,2-difluoro-D-ribofuranosyl-3,5-dibenzoate was added 10 ml of dichloromethane and 1.2 ml of triethylamine. The solution was cooled to -78°C and reacted with 0.57 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 23°C to form the titled blocked nucleoside which was confirmed by HPLC analysis. The beta to alpha anomeric ratio of the blocked nucleoside was 2.8:1. A quantitative HPLC analysis revealed a yield of blocked beta-anomer nucleoside of 50 percent.

### Example 17

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 112 ml of hexamethyldisilazane and 50 mg of ammonium sulfate. The mixture was heated to 115°C-120°C for 1 and 1/2 hours with stirring and the excess hexamethyldisilazane was subsequently removed. The resulting solid residue was reconstituted in 40 ml of dichloromethane at 23°C to form a homogenous bis-trimethylsilylcytosine solution.

To 1 g of 2-deoxy-2,2-difluoro-D-ribofuranosyl-3,5-dibenzoate was added 10 ml of dichloromethane and 0.54 ml of triethylamine. The solution was stirred at 23°C for 30 minutes, cooled to -78°C and reacted with 0.57 ml of trifluoromethanesulfonyl anhydride, in 0.50 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 23°C to form the titled blocked nucleoside which was confirmed by HPLC analysis. The beta to alpha anomeric ratio of the blocked nucleoside was 2.5:1. A quantitative HPLC analysis revealed a yield of blocked beta-anomer nucleoside of 68 percent.

### Example 18

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

To 5.78 g of cytosine were added 5 ml of dichloromethane, 20.6 ml of N-methyl-N-trimethylsilyltrifluoroacetamide and 5 ml of dichloromethane to form a homogenous bis-trimethylsilylcytosine solution.

To 1 g of 2-deoxy-2,2-difluoro-D-ribofuranosyl-3,5-dibenzoate was added 3 ml of dichloromethane and 0.55 ml of triethylamine. This solution was stirred at 23°C for 30 minutes, cooled to -78°C and reacted with 0.57 ml of trifluoromethanesulfonyl anhydride, in 1 ml of dichloromethane, to form alpha-anomer enriched 2-deoxy-2,2-difluoro-D-ribofuranosyl-3,5-dibenzoyl-1-trifluoromethanesulfonate in solution. Care was taken to maintain the temperature of the reaction mixture below -65°C. The alpha-anomer enriched 2-deoxy-2,2-difluoro-D-ribofuranosyl-3,5-dibenzoyl-1-trifluoromethanesulfonate solution was reacted with the bis-trimethylsilylcytosine solution at 23°C to form the titled blocked nucleoside which was confirmed by HPLC analysis. The beta to alpha anomeric ratio of the blocked nucleoside was 2.5:1.

To extract the nucleoside product from the reaction mixture, 250 ml of 1 N hydrochloric acid were added. The organic layer was separated and washed with 250 ml of 5% sodium carbonate. A quantitative HPLC analysis of the organic layer revealed a yield of blocked beta-anomer nucleoside of 50 percent.

The Table shows the effect the solvent, molar equivalents of pyrimidine nucleoside derivatives have on the anomeric ratio and yield of nucleoside product.

**Table**

| Solvent | (R') Base | (R') Base Equiv | Temp | α/β Nucleoside Ratio | β Yield |
|---|---|---|---|---|---|
| Dichloromethane | Cytosine | 20 | -25°C | 1:2.5 | 44% |
| Dichloromethane | Cytosine | 20 | -30°C | 1:2.3 | 45% |
| Dichloromethane | Cytosine | 20 | 0°C | 1:2.5 | 49% |
| Dichloromethane | Cytosine | 20 | 23°C | 1:2.2 | 49% |
| Dichloromethane & 1,2 Dichloroethane | Cytosine | 20 | 23°C | 1:1.8 | 31% |
| Dichloromethane & 1,2 Dichloroethane | Cytosine | 20 | 23°C | 1.1.9 | 42% |
| Dichloromethane & 1,2 Dichloroethane | Cytosine | 20 | 23°C | 1:2.8 | 50% |
| Dichloromethane | Cytosine | 20 | 23°C | 1:2.5 | 68% |
| Dichloromethane | Cytosine | 20 | -15°C | 1:2.3 | 58% |
| Dichloromethane | Cytosine | 20 | 27°C | 1:2.2 | 54% |
| Dichloromethane | Cytosine | 20 | 23°C | 1:2.2 | 49% |
| Dichloromethane | Cytosine | 30 | 10°C | 1:2.7 | 60% |
| Dichloromethane | Cytosine | 1.5 | 23°C | 1:1 | 17% |
| Dichloromethane & 1,2 Dichloroethane | Cytosine | 3 | 23°C | 1:1.3 | 6% |
| Dichloromethane & 1,2 Dichloroethane | Uracil | 2 | -20°C | 1:1 | N/D |
| Dichloromethane | Cytosine | 3.5 | -78°C | 1.3:1 | 10% |
| Dichloromethane & 1,2 Dichloroethane | N-Acetyl-Cytosine | 10 | -60°C | 1:2 | 15% |
| 1,2 Dichloroethane | Cytosine | 10 | -78°C | 1:3 | 28% |
| Dichloromethane | Cytosine | 10 | 0°C | 1:2.5 | 32% |
| Dichloromethane | 5-F-Uracil | 15 | 23°C | 1:1 | N/D |

The carbohydrate used to prepare the blocked nucleosides in the table was alpha-anomer enriched 2-deoxy-2,2-difluoro-D-ribofuranosyl-3,5-dibenzoyl-1-trifluoromethanesulfonate. (N/D) means not determined. The yields are based on the amount of carbohydrate and were calculated from a quantitative reverse phase HPLC analysis, wherein the corresponding solution product peak was compared with a standard. The protecting group for the above nucleoside base is trimethylsilyl.

### Example 19

### Preparation of beta-anomer enriched 1-(2'-deoxy-2',2'-difluoro-3',5'-di-O-benzoyl-D-ribofuranosyl)-4-aminopyrimidin-2-one

Cytosine (12.0 g), hexamethyldisilazane (60 ml) and ammonium sulfate (10 mg) were refluxed at 125°C for 30 minutes to form a homogenous solution. The hexamethyldisilazane was removed by distillation to form bis-trimethylsilylcytosine. 2-deoxy-2',2'-difluoro-D-ribofuranosyl-3,5-dibenzoyl-1-α-methanesulfonate (1.15 g) was reacted with the bis-trimethylsilylcytosine (6.89 g, 10 eq.) in anisole (2 ml) and acetonitrile (3 ml) at 80°C in the presence of the potassium salt of nanofluoro-1-butanesulfonic acid (0.5 g) for 16 hours. HPLC analysis confirmed completion of the reaction and indicated an in-situ yield of 33 percent. The beta to alpha anomer ratio of the titled compound was 3:1.

### Example 20

### Preparation of beta-anomer enriched 1-(2'-deoxy-2',2'-difluoro-3',5'-di-O-benzoyl-D-ribofuranosyl)-4-aminopyrimidin-2-one with potassium sulfate

2-deoxy-2,2-difluoro-D-ribofuranosyl-3,5-dibenzoyl-1-α-methanesulfonate (1.15 g) was reacted with the bis-trimethylsilylcytosine (6.89 g, 10 eq.) prepared as described in Example 19 in acetonitrile (2.0 ml) at 80°C in the presence of potassium sulfate (0.5 g) for 72 hours. HPLC analysis confirmed completion of the reaction and indicated an in-situ yield of 65 percent. The beta to alpha anomer ratio of the titled compound was 4.7:1.

### Example 21

### Preparation of beta-anomer enriched 1-(2'-deoxy-2',2'-difluoro-3',5'-di-O-benzoyl-D-ribofuranosyl)-4-aminopyrimidin-2-one with the tetrabutylammonium salt of trifluoromethanesulfonic acid

2-deoxy-2,2-difluoro-D-ribofuranosyl-3,5-dibenzoyl-1-α-methanesulfonate (0.29 ml) was reacted with the bis-trimethylsilylcytosine (6.89 g, 10 eq.) prepared as described in Example 19 in acetonitrile (3.0 ml) at 80°C in the presence of the tetrabutylammonium salt of trifluoromethanesulfonic acid (1.5 mmol) (prepared in-situ by treating tetrabutylammonium hydroxide (1.5 ml of a 1 molar solution in methanol) with trifluoromethanesulfonic acid (0.13 ml)), then distilling to remove the methanol) for 4 hours. HPLC analysis confirmed completion of the reaction and indicated an in-situ yield of 45 percent. The beta to alpha anomer ratio of the titled compound was 7.1:1

### Example 22

### Preparation of beta-anomer enriched 1-(2'-deoxy-2',2'-difluoro-3',5'-di-O-benzoyl-D-ribofuranosyl)-4-aminopyrimidin-2-one with barium sulfate

2-deoxy-2,2-difluoro-D-ribofuranosyl-3,5-dibenzoyl-1-α-methanesulfonate (1.15 g) was reacted with bis-trimethylsilylcytosine (6.89 g, 10 eq.) prepared as described in Example 19 in acetonitrile (3.0 ml) at 75°C in the presence of barium sulfate (1.0 g) for 20.5 hours. HPLC analysis indicated an in-situ yield of 36 percent. The beta to alpha anomer ratio of the titled compound was 11.2:1

### Example 23

### Preparation of beta-anomer enriched 1-(2'-deoxy-2',2'-difluoro-3',5'-di-O-benzoyl-D-ribofuranosyl)-4-aminopyrimidin-2-one with cesium sulfate

2-deoxy-2,2-difluoro-D-ribofuranosyl-3,5-dibenzoyl-1-α-methanesulfonate (1.15 g) was reacted with bis-trimethylsilylcytosine (6.89 g, 10 eq.) prepared as described in Example 19 in acetonitrile (3.0 ml) at 75°C in the presence of cesium sulfate (1.0 g) for 21 hours. HPLC analysis indicated an in-situ yield of 24 percent. The beta to alpha anomer ratio of the titled compound was 14.9:1

### Example 24

### Preparation of beta-anomer enriched 1-(2'-deoxy-2',2'-difluoro-3',5'-di-O-benzoyl-D-ribofuranosyl)-4-aminopyrimidin-2-one with the cesium salt of trifluoromethane sulfonic acid

2-deoxy-2,2-difluoro-D-ribofuranosyl-3,5-dibenzoy-1-α-methanesulfonate (1.15 g) was reacted with bis-trimethylsilylcytosine (6.89 g, 10 eq.) prepared as described in Example 19 in acetonitrile (3.0 ml) at 75°C in the presence of the cesium salt of trifluoromethanesulfonic acid (prepared in-situ by treating of 0.13 ml of trifluoromethane sulfonic acid with excess cesium carbonate) for 20.5 hours. HPLC analysis confirmed completion of the reaction and indicated an in-situ yield of 65 percent. The beta to alpha anomer ratio of the titled compound was 7.2:1

### Example 25

### Preparation of beta-anomer enriched 1-(2'-deoxy-2',2'-difluoro-3',5'-di-O-benzoyl-D-ribofuranosyl)-4-aminopyrimidin-2-one with the barium salt of trifluoromethanesulfonic acid

2-deoxy-2,2-difluoro-D-ribofuranosyl-3,5-dibenzoyl-1-α-methanesulfonate (1.15 g) was reacted with bis-trimethylsilylcytosine (6.89 g, 10 eq.) prepared as described in Example 19 in acetonitrile (3.0 ml) at 75°C in the presence of the barium salt of trifluoromethanesulfonic acid (prepared in-situ by treating of 0.13 ml of trifluoromethanesulfonic acid with excess barium carbonate) for 20.5 hours. HPLC analysis indicated an in-situ yield of 25 percent. The beta to alpha anomer ratio of the titled compound was 14.4:1

### Example 26

### Preparation of beta-anomer enriched 1-(2'-deoxy-2',2'-difluoro-3',5'-di-O-benzoyl-D-ribofuranosyl)-4-aminopyrimidin-2-one with the potassium salt of trifluoromethanesulfonic acid

2-deoxy-2,2-difluoro-D-ribofuranosyl-3,5-dibenzoyl-1-α-methanesulfonate (2.3 g, 12.6 eq.) was reacted with bis-trimethylsilylcytosine (16.1 g) prepared as described in Example 19 in acetonitrile (8.0 ml) at 75°C and in the presence of the potassium salt of trifluoromethanesulfonic acid (prepared in-situ by treating trifluoromethanesulfonic acid (0.26 ml) with potassium carbonate (1.0 g)) for 45 hours. HPLC analysis indicated an in-situ yield of 69.8 percent. The beta to alpha anomer ratio of the titled compound was 7.2:1.

To extract the nucleoside product, the reaction mixture was cooled between 70°C and 80°C and combined with 40 ml of 4 N hydrochloric acid. The product precipitated, was filtered, and dried. A quantitative HPLC analysis indicated an isolated yield of 62.4 percent.

### Example 27

### Preparation of beta-anomer enriched 1-(2'-deoxy-2',2'-difluoro-3',5'-di-O-benzoyl-D-ribofuranosyl)-4-aminopyrimidin-2-one with the potassium salt of trifluoromethanesulfonic acid

2-deoxy-2,2-difluoro-D-ribofuranosyl-3,5-dibenzoyl-1-α-methanesulfonate (2.3 g) was reacted with bis-trimethylsilylcytosine (16.1 g, 12.6 eq.) prepared as described in Example 19 in propionitrile (8.0 ml) at 90°C and in the presence of the potassium salt of trifluoromethanesulfonic acid (prepared in-situ by treating trifluoromethanesulfonic acid (0.26 ml) with potassium carbonate (1.0 g)) for 21 hours. HPLC analysis confirmed completion of the reaction. The beta to alpha anomer ratio of the titled compound was 6.7:1.

To extract the nucleoside product, the reaction mixture was cooled between 70°C and 80°C and combined with 40 ml of 4 N hydrochloric acid. The product precipitated, was filtered, and dried. A quantitative HPLC analysis indicated an isolated yield of 59.3 percent.

### Comparative Example 28

### Preparation of beta-anomer enriched 1-(2'deoxy-2',2'-difluoro-3',5'-di-O-benzoyl-D-ribofuranosyl)-4-aminopyrimidin-2-one without a catalyst

2-deoxy-2,2-difluoro-D-ribofuranosyl-3,5-dibenzoyl-1-α-methanesulfonate (1.15 g) was reacted with bis-trimethylsilylcytosine (6.09 g, 10 eq.) prepared as described in Example 19 in anisole (4 ml) at 110°C for 20 hours. HPLC analysis confirmed completion of the reaction and indicated an in-situ yield of 77 percent. The beta to alpha anomer ratio of the titled compound was 3.4:1.

### Comparative Example 29

### Preparation of beta-anomer enriched 1-(2'deoxy-2',2'-difluoro-3',5'-di-O-benzoyl-D-ribofuranosyl)-4-aminopyrimidin-2-one without a catalyst

2-deoxy-2,2-difluoro-D-ribofuranosyl-3,5-dibenzoyl-1-α-methanesulfonate (1.15 g) was reacted with bis-trimethylsilylcytosine (6.08 g, 10 eq.) prepared as described in Example 19 in propionitrile (4 ml) at 85°C in the presence of the cesium salt of trifluoromethanesulfonic acid (prepared in-situ by treating trifluoromethanesulfonic acid (0.13 ml) with excess cesium carbonate) for 20 hours. HPLC analysis confirmed completion of the reaction and indicated an in-situ yield of 70 percent. The beta to alpha anomer ratio of the titled compound was 6.7:1.

### Example 30

### Preparation of beta-anomer enriched 9-[1-(2'-deoxy-2',2'-difluoro-3',5'-di-O-benzoyl-D-ribofuranosyl)]-2,6-dipivalamidopurine with 2 equivalents of 2,6-dipivalamidopurine potassium salt

To 100 mg of 2-deoxy-2,2-difluoro-D-ribofuranosyl-3,5-dibenzoate were added 1 ml of dichloromethane and 0.036 ml of triethylamine. This solution was stirred at 23°C for 15 minutes, cooled to -40°C and reacted with 0.045 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 185 mg suspension of 2,6-dipivalamidopurine was prepared in 1.5 ml of acetonitrile and maintained anhydrous under a nitrogen atmosphere. 65 mg of potassium t-butoxide were added to the suspension and the resulting mixture was stirred at 23°C for 10 minutes to form a 2,6-dipivalamidopurine potassium salt. The salt was cooled, to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analysis. The beta to alpha anomeric ratio of the blocked nucleoside was 2:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. A quantitative HPLC analysis revealed a combined yield of blocked beta- and alpha-anomer nucleoside of 42 percent.

### Example 31

### Preparation of beta-anomer enriched 9-[1-(2'-deoxy-2',2'-difluoro-3',5'-di-O-benzoyl-D-ribofuranosyl)]-2,6-dipivalamidopurine with 2 equivalents of 2,6-dipivalamidopurine potassium salt

To 1 g of 2-deoxy-2,2-difluoro-D-ribofuranosyl-3,5-dibenzoate was added 0.55 ml of triethylamine and 8.33 ml of dichloromethane at 23°C. The mixture was cooled to -78°C and reacted with 0.53 ml of trifluoromethanesulfonic anhydride in 0.50 ml of dichloromethane to form alpha-anomer enriched 2-deoxy-2,2-difluoro-D-ribofuranosyl-3,5-dibenzoate-1-trifluoromethanesulfate in solution. Care was taken to maintain the temperature of the reaction mixture below -65°C.

A 1.85 g suspension of 2,6-dipivalamidopurine was prepared in 30 ml of acetonitrile and maintained anhydrous under a nitrogen atmosphere. 651 mg of potassium t-butoxide were added to the suspension and the resulting mixture stirred at 23°C for 15 minutes to form a 2,6-dipivalamidopurine potassium salt. The salt suspension was added to 20 ml of dry dichloromethane, cooled to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 23°C to form the titled blocked nucleoside which was confirmed by HPLC analysis. The beta to alpha anomeric ratio of the blocked nucleoside was 2:1.

To extract the nucleoside product from the reaction mixture, 50 ml of ethyl acetate and 50 ml of 1 N hydrochloric acid were added. The organic layer was separated and washed with 50 ml of 5% sodium bicarbonate. The organic layer was separated and washed with 50 ml of saturated aqueous sodium chloride and dried over magnesium sulfate.

### Example 32

### Preparation of beta-anomer enriched 9-[1-(2'-deoxy-2',2'-difluoro-3',5'-di-O-benzoyl-D-ribofuranosyl)]-6-chloropurine with 2 equivalents of 6-chloropurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 155 mg suspension of 6-chloropurine was prepared in 3 ml of acetonitrile and maintained anhydrous under a nitrogen atmosphere. 130 mg of potassium t-butoxide were added to the suspension and the resulting mixture was stirred at 23°C for 10 minutes to form a 6-chloropurine potassium salt. The salt suspension was cooled to 0°C and reacted with 2 ml of the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analysis. The beta to alpha anomeric ratio of the blocked nucleoside was 2:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution and 5 ml of brine and dried over magnesium sulfate. A quantitative HPLC analysis revealed a yield of blocked beta-anomer nucleoside of 27 percent.

### Example 33

### Preparation of beta-anomer enriched 9-[1-(2'-deoxy-2',2'-difluoro-3',5'-di-O-benzoyl-D-ribofuranosyl)]-2,6-dichloro-3-deazapurine with 2 equivalents of 2,6-dichloro-3-deazapurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 82 mg suspension of 2,6-dichloro-3-deazapurine was prepared in 1.5 ml of acetonitrile and maintained anhydrous under a nitrogen atmosphere. 49 mg of potassium t-butoxide were added to the suspension and the resulting mixture was stirred at 23°C for 10 minutes to form a 2,6-dichloro-3-deazapurine potassium salt. The salt suspension was cooled to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 20°C to form the titled blocked nucleoside which was confirmed by HPLC analysis. The beta to alpha anomeric ratio of the blocked nucleoside was 2.5:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution and 5 ml of brine and dried over magnesium sulfate. A quantitative HPLC analysis revealed a combined yield of blocked beta-anomer nucleoside of 21 percent, mp 127°C-129°C.

### Example 34

### Preparation of beta-anomer enriched 9-[1-(2'-deoxy-2',2'-difluoro-3',5'-di-O-benzoyl-D-ribofuranosyl)]-2,6-dichloropurine with 2 equivalent of 2,6-dichloropurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes then cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 220 mg suspension of 2,6-dichloropurine was prepared in 3 ml of acetonitrile and maintained anhydrous under a nitrogen atmosphere. 130 mg of potassium t-butoxide were added and the resulting mixture was stirred at 23°C for 10 minutes to form a 2,6-dichloropurine potassium salt. The salt suspension was cooled to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analysis. The beta to alpha anomeric ratio of the blocked nucleoside was 2.5:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. A quantitative HPLC analysis revealed a yield of blocked beta-anomer nucleoside of 22 percent.

### Example 35

### Preparation of beta-anomer enriched 1-[1-(2'-deoxy-2',2'-difluoro-3',5'-di-O-benzoyl-D-ribofuranosyl)]-3-carboethoxy-1,2,4-triazole with 2 equivalents of 3-carboethoxy-1,2,4-triazole potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 164 mg suspension of triazole ester was prepared in 3 ml of acetonitrile and maintained anhydrous under a nitrogen atmosphere. 131 mg of potassium t-butoxide were added and the resulting mixture was stirred at 23°C for 10 minutes to form a 3-carboethoxy-1,2,4-triazole potassium salt. The salt suspension was cooled to 0°C and reacted with 2 ml of the alpha-anomer enriched 2-deoxy-2,2-difluoro-D-ribofuranosyl-3,5-dibenzoyl-1-trifluoromethanesulfonate solution, stirred for 40 minutes and warmed to 15°C to form the titled blocked nucleoside which was confirmed by HPLC analysis. The beta to alpha anomeric ratio of the blocked nucleoside was 2.5:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. A quantitative HPLC analysis revealed a yield of blocked beta-anomer nucleoside of 14 percent.

### Example 36

### Preparation of beta-anomer enriched 9-[1-(2'-deoxy-2',2'-difluoro-3',5'-di-O-benzoyl-D-ribofuranosyl)]-2-amino-6-chloropurine with 2 equivalents of 2-amino-6-chloropurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 197 mg suspension of 2-amino-6-chloropurine was prepared in 3 ml of acetonitrile and maintained anhydrous under a nitrogen atmosphere. 130 mg of potassium t-butoxide were added and the resulting mixture was stirred at 23°C for 10 minutes to form a 2-amino-6-chloropurine potassium salt. The salt suspension was cooled to 0°C and reacted with 2 ml of the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analyses. The beta to alpha anomeric ratio of the blocked nucleoside was 2:1.

To extract the nucleoside product from the reaction mixture, 100 ml of ethyl acetate, 10 ml of water were added and a precipitate formed. The precipitate was filtered off, washed with 5 ml of saturated aqueous sodium bicarbonate solution, 5 ml of brine and dried over magnesium sulfate. A quantitative HPLC analysis revealed a yield of blocked beta-anomer nucleoside of 14 percent.

### Example 37

### Preparation of beta-anomer enriched 9-[1-(2'-deoxy-2',2'-difluoro-3',5'-di-O-benzoyl-D-ribofuranosyl)]-2,6-dipivalamidopurine with 2 equivalents of 2,6-dipivalamidopurine potassium salt

To 3.78 g of 2-deoxy-2,2-difluoro-D-ribofuranosyl-3,5-dibenzoate were added 30 ml of dichloromethane and 1.39 ml of triethylamine. This solution was stirred at 23°C for 15 minutes, cooled to -40°C and reacted with 1.68 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 6.99 g suspension of 2,6-dipivalamidopurine was prepared in 100 ml of acetonitrile and maintained anhydrous under a nitrogen atmosphere. 2.46 g of potassium t-butoxide were added and the resulting mixture was stirred at 23°C for 10 minutes and dried to constant weight in vacuo at 40°C to form a 2,6-dipivalamidopurine potassium salt. The salt suspension was added to 100 ml dichloromethane, cooled to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analysis.

To extract the nucleoside product from the reaction mixture, 500 ml of ethyl acetate, 20 ml of ice, 20 ml of 1 N hydrochloric acid and 35 ml of a saturated aqueous sodium chloride solution were added. The organic layer was separated, washed with 25 ml of saturated aqueous sodium bicarbonate solution, 25 ml of brine and dried over magnesium sulfate. The beta to alpha anomeric ratio of the blocked nucleoside was 1.8:1. A quantitative HPLC analysis revealed a yield of blocked beta-anomer nucleoside of 28 percent, mp 238°C-239°C.

### Example 38

### Preparation of beta-anomer enriched 9-[1-(2'-deoxy-2',2'-difluoro-3',5'-di-O-benzoyl-D-ribofuranosyl)]-6-pivalamidopurine with 2 equivalents of 6-pivalamidopurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A 255 mg suspension of 6-pivalamidopurine was prepared in 3 ml of acetonitrile and maintained anhydrous under a nitrogen atmosphere. 131 mg of potassium t-butoxide were added and the resulting mixture was stirred at 23°C for 10 minutes to form a 6-pivalamidopurine potassium salt. The salt suspension was cooled to 0°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 22°C to form the titled blocked nucleoside which was confirmed by HPLC analysis. The beta to alpha anomeric ratio of the blocked nucleoside was 2:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. A quantitative HPLC analysis revealed a combined yield of blocked beta- and alpha-anomer nucleoside of 28 percent.

### Example 39

### Preparation of beta-anomer enriched 9-[1-(2'-deoxy-2',2'-difluoro-3',5'-di-O-benzoyl-D-ribofuranosyl)]-8-bromo-7-cyano-7-deaza-6-pivalamidopurine with 2 equivalents of 8-bromo-7-cyano-7-deaza-6-pivalamidopurine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2-deoxy-2,2-difluoro-D-ribofuranosyl-3,5-dibenzoyl-1-trifluoromethanesulfonate in solution.

A suspension of 187 mg of 8-bromo-7-cyano-7-deaza-6-pivalamidopurine was prepared in 3 ml of acetonitrile and maintained anhydrous under a nitrogen atmosphere. 65 mg of potassium t-butoxide were added and the resulting mixture was stirred at 23°C for 10 minutes to form a 8-bromo-7-cyano-7-deaza-6-pivalamidopurine potassium salt. The salt suspension was cooled to 0°C and reacted with 1 ml of the alpha-anomer enriched 2-deoxy-2,2-difluoro-D-ribofuranosyl-3,5-dibenzoyl-1-trifluoromethanesulfonate solution, stirred for 1 hour and warmed to 20°C to formed the titled blocked nucleoside which was confirmed by HPLC analysis. The beta to alpha anomeric ratio of the blocked nucleoside was 2:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. A quantitative HPLC analysis revealed a combined yield of blocked beta- and alpha-anomer nucleoside of 24 percent.

### Example 40

### Preparation of beta-anomer enriched 9-[1-(2'-deoxy-2',2'-difluoro-3',5'-di-O-benzoyl-D-ribofuranosyl)]-2,6-dipivalamidopurine with 2 equivalents of 2,6-dipivalamidopurine potassium salt in various reaction solvents

To 1 g of 2-deoxy-2,2-difluoro-D-ribofuranosyl-3,5-dibenzoate was added 10 ml of dichloromethane and 0.36 ml of triethylamine. This solution was stirred at 23°C for 15 minutes, cooled to -40°C and reacted with 0.45 ml of trifluoromethanesulfonic anhydride to form alpha-anomer enriched 2-deoxy-2,2-difluoro-D-ribofuranosyl-3,5-dibenzoyl-l-trifluoromethanesulfonate in solution.

A 1.85 g suspension of 2,6-dipivalamidopurine was prepared in 30 ml of acetonitrile and maintained anhydrous under a nitrogen atmosphere. 0.65 g of potassium t-butoxide were added and the resulting mixture was stirred at 25°C for 10 minutes to form a 2,6-dipivalamidopurine potassium salt. The salt suspension was dried in vacuo at 40°C to form a white solid of constant weight. 207 mg of the purine salt was suspended in 1.5 ml of the solvent shown in Runs A-F in the table below, under nitrogen at 0°C and reacted with 1 ml of the alpha-anomer enriched 2-deoxy-2,2-difluoro-D-ribofuraosyl-3,5-dibenzoyl-1-trifluoromethanesulfonate solution, stirred for 1 hour and warmed to 0°C to form the titled blocked nucleoside which was confirmed by HPLC analysis. The beta to alpha anomeric ratios of the blocked nucleoside is shown below.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 2 ml of saturated aqueous sodium bicarbonate solution, 5 ml of brine and dried over magnesium sulfate. A quantitative HPLC analysis revealed a yield of blocked beta-anomer nucleoside shown below:

| RUN | Solvent | β/α Nucleoside Ratio | β Yield (%) |
|---|---|---|---|
| A | Tetrahydrofuran | 1.3:1 | 45 |
| B | Toluene | 1.8:1 | 49 |
| C | Ethylacetate | 1.6:1 | 47 |
| D | Dichloroethane | 2.1:1 | 55 |
| E | t-Butylalcohol | 3.5:1 | 53 |
| F | Acetonitrile | 1.6:1 | 40 |

### Example 41

### Preparation of beta-anomer enriched 9-[1-(2'-deoxy-2',2'-difluoro-3',5'-di-O-benzoyl-D-ribofuranosyl)]-2-acetamido-6-diphenylcarbamoyloxypurine with 2 equivalents of 2-acetamido-6-diphenylcarbamoyloxy purine potassium salt

To 1.4 g of 2-deoxy-2,2-difluoro-D-ribofuranosyl-3,5-dibenzoate were added 14 ml of dichloromethane and 0.515 ml of triethylamine. This solution was stirred at 23°C for 15 minutes, cooled to -40°C and reacted with 0.621 ml of trifluoromethanesulfonic anhydride to form alpha-anomer enriched 2,2-difluoro-2-deoxy-D-ribofuranosyl-3,5-dibenzoyl-1-trifluoromethanesulfonate in solution.

A solution of 2.56 g 2-acetamido-6-diphenylcarbamoyloxypurine was prepared in 50 ml of hot dimethylformamide and maintained anhydrous under a nitrogen atmosphere. The solution was cooled to 25°C and 0.74 g of potassium t-butoxide were added. The resulting mixture was stirred at 23°C for 10 minutes and evaporated to an oil which was triturated with ether, collected on a filter, and dried in vacuo at 40°C to form a 2-acetamido-6-diphenyl carbamoyloxy purine potassium salt. 496 mg of the purine salt were suspended in 3 ml dichloromethane, cooled to 5°C and reacted with the alpha-anomer enriched 2-deoxy-2,2-difluoro-D-ribofuranosyl-3,5-dibenzoyl-1-trifluoromethanesulfonate solution, stirred for 1 hour and warmed to 25°C to form the titled blocked nucleoside which was confirmed by HPLC analysis. The beta to alpha anomeric ratio of the blocked nucleoside was 1.8:1.

To extract the nucleoside product from the reaction mixture, 25 ml of ethyl acetate, 1 ml of water, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. The yield of blocked beta-anomer nucleoside was 5.8 percent.

### Example 42

### Preparation of beta-anomer enriched 9-[1-(2'-deoxy-2',2'-difluoro-3',5'-di-O-benzoyl-D-ribofuranosyl)]-2,6-dipivalamidopurine with 7 equivalents of 2,6-dipivalamidopurine potassium salt

To 100 mg of 2-deoxy-2,2-difluoro-D-ribofuranosyl-3,5-dibenzoate were added 3 ml of dichloromethane and 0.036 ml of triethylamine. This solution was stirred at 23°C for 15 minutes, cooled to -78°C and reacted with 0.045 ml of trifluoromethanesulfonic anhydride to form alpha-anomer enriched 2-deoxy-2,2-difluoro-D-ribofuranosyl-3,5-dibenzoyl-1trifluoromethanesulfonate in solution.

A 1.85 g suspension of 2,6-dipivalamidopurine was prepared in 30 ml of acetonitrile and maintained anhydrous under a nitrogen atmosphere. 0.65 g of potassium t-butoxide were added and the resulting mixture was stirred at 23°C for 10 minutes and dried in vacuo at 40°C to form a 2,6-dipivalamidopurine potassium salt which was cooled to -78°C. The purine salt were reacted with the alpha-anomer enriched 2-deoxy-2,2-difluoro-D-ribofuranosyl-3,5-dibenzoyl-1-trifluoromethanesulfonate solution at 23°C, stirred for 1.5 hours and warmed to 22°C to form the titled blocked nucleoside which was confirmed by HPLC analysis.

To extract the nucleoside product from the reaction mixture, 25 ml of ethyl acetate, 1 ml of ice, 1 ml of 1 N hydrochloric acid and 2 ml of a saturated aqueous sodium chloride solution were added. The organic layer was separated, washed with 5 ml of saturated aqueous sodium bicarbonate solution, 5 ml of brine and dried over magnesium sulfate. The beta to alpha anomeric ratio of the blocked nucleoside was 2.7:1.

### Example 43

### Preparation of beta-anomer enriched 1-(2'-deoxy-2',2'-difluoro-3',5'-di-O-benzoyl-D-ribofuranosyl)-4-aminopyrimidin-2-one with 3 equivalents of bis-trimethylsilylcytosine

Bis-trimethylsilylcytosine was prepared by combining 292 mg of cytosine with 2 ml of hexamethyldisilazane, 11 mg of ammonium sulfate and 5 ml of xylenes and refluxing the solution for one hour to form a homogenous solution. The excess xylenes and hexamethyldisilazane were removed leaving behind a molten residue of bis-trimethylsilylcytosine. 400 mg of 2-deoxy-2,2-difluoro-D-ribofuranosyl-3,5-dibenzoyl-1-α -methanesulfonate, dissolved in 2 ml of xylenes, were added to the molten bis-trimethylsilylcytosine and the xylenes were removed. The temperature of the reaction mixture was maintained at 160°C for 15 minutes. HPLC analysis confirmed completion of the reaction. The alpha to beta anomeric ratio of blocked nucleoside product was 1:1.3.

To extract the nucleoside product, the reaction mixture was cooled, diluted in 50 ml of ethyl acetate and washed with 50 ml of 1 N hydrochloric acid.

### Example 44

### Preparation of beta-anomer enriched 1-(2'-deoxy-2',2'-difluoro-3',5'-di-O-benzoyl-D-ribofuranosyl)-4-ketopyrimidin-2-one with 3 equivalents of bis-trimethylsilyluracil

Bis-trimethylsilyluracil was prepared by combining 295 mg of uracil with 5 ml of hexamethyldisilazane, 11 mg of ammonium sulfate and 10 ml of 1,2-dichloroethane. The solution was heated to 110°C for one hour to form a homogenous solution and the excess xylenes and hexamethyldisilazane were removed to form molten bis-trimethylsilyluracil. 200 mg of 2-deoxy-2,2-difluoro-D-ribofuranosyl-3,5-dibenzoyl-1-α -methanesulfonate were added to the molten bis-trimethylsilyluracil. The temperature of the reaction mixture was maintained at 150°C for 2 hours. HPLC analysis confirmed completion of the reaction. The alpha to beta anomeric ratio of blocked nucleoside product was 1:1.8.

To extract the nucleoside product, the reaction mixture was cooled, diluted in 50 ml of ethyl acetate and washed with 50 ml of 1 N hydrochloric acid.

### Example 45

### Preparation of beta-anomer enriched 1-(2'-deoxy-2',2'-difluoro-3',5'-di-O-benzoyl-D-ribofuranosyl)-4-aminopyrimidin-2-one with 10 equivalents of bis-trimethylsilylcytosine

To 1.12 g of molten bis-trimethylsilylcytosine were added 200 mg of 2-deoxy-2,2-difluoro-D-ribofuranosyl-3,5-dibenzoyl-1-α-methanesulfonate. The temperature of the reaction mixture was maintained at 130°C for 1 hour. HPLC analysis confirmed completion of the reaction. The anomeric ratio of the blocked nucleoside product was 1.7:1 beta to alpha.

To extract the nucleoside product, the reaction mixture was diluted with 100 ml ethyl acetate and washed with 100 ml of 1 N hydrochloric acid. A quantitative HPLC analysis of the organic layer indicated that the yield of blocked beta-anomer nucleoside was 50 percent.

### Example 46

### Preparation of beta-anomer enriched 1-(2'-deoxy-2',2'-difluoro-3',5'-di-O-benzoyl-D-ribofuranosyl)-4-acetamidopyrimidin-2-one with 3 equivalents of bis-trimethylsilyl-N-acetylcytosine

To 500 mg of bis-trimethylsilyl-N-acetylcytosine were added 980 mg of 2-deoxy-2,2-difluoro-D-ribofuranosyl-3,5-dibenzoyl-1-α-methanesulfonate. The temperature of the reaction mixture was maintained at 108°C for 3 hours. HPLC analysis confirmed completion of the reaction. The beta to alpha anomeric ratio of the blocked nucleoside product was 1.4:1.

To extract the nucleoside product, the reaction mixture was cooled, diluted with 25 ml ethyl acetate and washed with 25 ml of 1 N hydrochloric acid. The aqueous layer was washed with 30 ml of ethyl acetate. A quantitative HPLC analysis of the ethyl acetate layer indicated that the yield of blocked beta-anomer nucleoside was 34 percent.

### Example 47

### Preparation of beta-anomer enriched 1-(2'-deoxy-2',2'-difluoro-3',5'-di-O-benzoyl-D-ribofuranosyl)-4-acetamidopyridin-2-one with 3 equivalents of bis-trimethylsilyl-N-acetylcytosine

To 393 mg of molten bis-trimethylsilyl-N-acetylcytosine were added 200 mg of 2-deoxy-2,2-difluoro-D-ribofuranosyl-3,5-dibenzoyl-1-α-methanesulfonate. The temperature of the reaction mixture was maintained at 110°C for 1 hour. The beta to alpha anomeric ratio of the blocked nucleoside product was 2.3:1.

To extract the nucleoside product, the reaction mixture was diluted with 40 ml ethyl acetate and washed with 25 ml of 1 N hydrochloric acid. A quantitative HPLC analysis of the organic layer indicated that the yield of beta-anomer nucleoside was 27 percent.

### Example 48

### Preparation of beta-anomer enriched 1-(2'-deoxy-2',2'-difluoro-3',5'-di-O-benzoyl-D-ribofuranosyl)-4-aminopyrimidin-2-one with 20 equivalents of bis-trimethylsilylcytosine

Bis-trimethylsilylcytosine was prepared by combining 4.9 g of cytosine with 90 ml of hexamethyldisilazane, 581 mg of ammonium sulfate and 2 ml of xylenes and heating the solution for two hours to form a homogenous solution. The excess hexamethyldisilazane was removed and a white residue formed. 1 g of 2-deoxy-2,2-difluoro-D-ribofuranosyl-3,5-dibenzoyl-1-α -methanesulfonate, dissolved in 5 ml of acetonitrile, was added to the bis-trimethylsilylcytosine solution and the acetonitrile removed. The temperature of the reaction mixture was maintained at 130°C under vacuum for 1 hour. HPLC analysis confirmed completion of the reaction. The beta to alpha anomeric ratio of the blocked nucleoside product was 3.9:1.

To extract the nucleoside product, the reaction mixture was diluted with 100 ml dichloromethane and washed sequentially with 100 ml of 1 N hydrochloric acid and 200 ml of 5% sodium bicarbonate followed by 200 ml of saturated sodium chloride. The organic layer was dried over magnesium sulfate, filtered and evaporated to 1.03 g of a yellow solid. A quantitative HPLC analysis indicated that the yield of beta-anomer nucleoside was 43 percent.

The following Table shows how the carbohydrate selected, reaction temperature and molar equivalents of nucleobase effect the yield and anomeric ratio of the nucleoside product.

**Table**

| Carbo. | Base (R') | Base (R') Equiv. | Temp. | α/β Nucleoside Ratio | Yield |
|---|---|---|---|---|---|
| 1:1 α:β-OMs | Cytosine | 1.5 | 130°C | 3:1 | N/D |
| α-OMs | Cytosine | 3.0 | 160°C | 1:1.3 | N/D |
| α-OMs | Cytosine | 10.0 | 130°C | 1:1.7 | 50% β |
| α-OMs | Uracil | 3.0 | 150°C | 1:1.8 | N/D |
| α-OMs | N-Acetyl-Cytosine | 3.0 | 115°C | 1:1.4 | 34% β |
| α-OMs | N-Acetyl-cytosine | 3.0 | 110°C | 1:2.3 | 27% β |
| α-OMs | Cytosine | 20.0 | 130°C | 1:4 | 43% β |

(N/D) means not determined. The carbohydrates (carbo.) are hydroxy protected. α - or β -OMs is alpha- or beta-2,2-difluoro-2-deoxy-D-ribofuranosyl-3,5-dibenzoyl-1-methanesulfonate and β -or α -OTs is beta- or alpha- 2,2-difluoro-2-deoxy-D-ribofuranosyl-3,5-dibenzoyl-1-toluenesulfonate. The yields are based on the total amount of carbohydrate and were calculated from a quantitative reverse phase HPLC analysis, wherein the corresponding solution product peak was compared with a standard, 1-(2'-deoxy-2',2'-difluoro-3',5'-di-O-benzoyl-beta-D-ribofuranosyl)-4-aminopyrimidin-2-one. The nucleoside base protecting group in each example is trimethylsilyl.

### Example 49

### Preparation of beta-anomer enriched 1-(2'-deoxy-2',2'-difluoro-3',5'-di-O-benzoyl-D-ribofuranosyl)-4-pivalamidopyrimid-2-one in acetonitrile

N-pivalamidocytosine (1.0 g, 5.5 mmol) was suspended in acetonitrile (15.0 ml) and treated with potassium t-butoxide (0.062 g, 5.5 mmol) and stirred under a nitrogen atmosphere at 25°C to form the potassium salt of N-pivaloyl cytosine.

2-deoxy-2,2-difluoro-D-ribofuranosyl-3,5-dibenzoyl-1-α-(p-bromobenzene)sulfonate (2.99 g, 5.0 mmol), in acetonitrile (10.0 ml), was added to the above salt and the entire mixture was reacted for 5.5 hours at 65°C to form a blocked nucleoside. HPLC analysis confirmed completion of the reaction and indicated a beta to alpha anomeric ratio of 3.9:1.

To isolate the nucleoside product, the reaction mixture was distributed between with ethyl acetate and water and the organic layer was washed with sodium bicarbonate and dried over magnesium sulfate. Column chromatography (silica gel, toluene/ethyl acetate 6:4) gave 0.700 g of the titled product at a yield of 20 percent; m.p. 191°C-193°C.

### Example 50

### Preparation of beta-anomer enriched 1-(2'-deoxy-2',2'-difluoro-3',5'-di-O-benzoyl-D-ribofuranosyl)-4-pivalamido)pyrimid-2-one in acetonitrile

N-pivaloylcytosine (0.098 g, 0.5 mmol) was suspended in acetonitrile (1.5 ml) and treated with potassium t-butoxide (0.062 g, 0.55 mmol) and stirred under a nitrogen atmosphere at 25°C to form the potassium salt of N-pivaloylcytosine.

2-deoxy-2,2-difluoro-D-ribofuranosyl-3,5-dibenzoyl-1-α-iodide (0.244 g, 0.5 mmol), in acetonitrile (1.5 ml), was added to the above salt and the entire mixture was reacted for 24 hours at 60°C to form a blocked nucleoside. HPLC analysis confirmed completion of the reaction and indicated a beta to alpha anomeric ratio of 1.13:1.

### Example 51

### Preparation of beta-anomer enriched 1-(2'-deoxy-2',2'-difluoro-3',5'-di-O-benzoyl-D-ribofuranosyl)-1,2,4-triazole-3-carbonitrile in acetonitrile

1,2,4-triazole-3-carbonitrile (0.101 g, 1.03 mmol) was suspended in acetonitrile (10 ml) and treated with sodium hydride (0.0445 g, 1.12 mmol) and stirred under a nitrogen atmosphere at 25°C to form the corresponding sodium salt of the triazole. 2-deoxy-2,2-difluoro-D-ribofuranosyl-3,5-dibenzoyl-1-α-bromide (0.451 g, 1.02 mmol), in acetonitrile (10 ml), was added to the above salt and the entire mixture was reacted for 78 hours at 82°C to form a blocked nucleoside. HPLC analysis confirmed completion of the reaction and indicated a beta to alpha anomeric ratio of 1.2:1.

To isolate the nucleoside product, the reaction mixture was evaporated to from an oily solid, diluted with ethyl acetate, washed with sodium bicarbonate and dried over magnesium sulfate and concentrated. The residue crystallized from ethanol to give 30 mg of a titled product at a yield of 6 percent; m.p. 225°C-226°C. MS(FD) M/Z 455 (M+1) Elemental Analysis for C₂₂H₁₆F₂N₄O₅: (Theoretical) C, 58.15; H, 3.55; N, 12.33; (Empirical) C, 58.36; H, 3.79; N, 12.10.

### Example 52

### Preparation of beta-anomer enriched 1-(2'-deoxy-2',2'-difluoro-3',5'-di-O-benzoyl-D-ribofuranosyl)-1,2,4-triazole-3-carbonitrile in acetonitrile

1,2,4-triazole-3-carbonitrile (0.272 g, 2.89 mmol) was suspended in acetonitrile (20 ml), treated with sodium hydride (0.094 g, 2.7 mmol) and stirred under a nitrogen atmosphere at 25°C to form the sodium salt of the triazole.

2-deoxy-2,2-difluoro-D-ribofuranosyl-3,5-dibenzoyl-1-α-iodide (0.941 g, 1.9 mmol), in acetonitrile (20 ml), was added to the above salt and the entire mixture was reacted for 48 hours at 82°C to form a blocked nucleoside. HPLC analysis confirmed completion of the reaction and indicated a beta to alpha anomeric ratio of 3.5:1.

To isolate the nucleoside product, the reaction mixture was evaporated to from an oily solid, diluted with ethyl acetate, washed with sodium bicarbonate, dried over magnesium sulfate and concentrated. The residue crystallized from ethanol to give 0.421 g of the titled product; m.p. 225°C-226°C at a yield of 48 percent. MS(FD) M/Z 455 (M+1) Elemental Analysis for C₂₂H₁₆F₂N₄O₅: (Theoretical) C, 58.15; H, 3.55; N, 12.33; (Empirical) C, 58.35; H, 3.65; N, 12.33.

### Example 53

### Preparation of (9)regioisomer-beta-anomer enriched 1-(2'-deoxy-2',2'-difluoro-3',5'-di-O-benzoyl-D-ribofuranosyl)-6-cyanopurine in N,N-dimethylacetamide

6-cyanopurine (0.92 g, 6.35 mmol) was suspended in N,N-dimethylacetamide (12 ml) and treated with sodium hydride (0.396 g, 8.25 mmol) and stirred under a nitrogen atmosphere at 25°C to form the sodium salt of 6-cyanopurine.

2-deoxy-2,2-difluoro-D-ribofuranosyl-3,5-dibenzoyl-1-α-iodide (3.09 g, 6.35 mmol), in N,N-dimethylacetamide(4 ml), was added to the above salt and the entire mixture was reacted for 5 hours at 70°C to form a blocked nucleoside. HPLC analysis confirmed completion of the reaction and indicated a beta to alpha anomeric ratio of 1.2:1.

To isolate the nucleoside product, the reaction mixture was cooled, the solvent removed under vacuum, the residue was dissolved in ethyl acetate, washed with a 0.2 M lithium chloride solution, dried over magnesium sulfate and concentrated. Column chromatography (silica gel, toluene/ethyl acetate 9:1) gave 0.21 g of the titled product at a yield of 6.5 percent. MS(FD) 506 (M+1) Elemental Analysis for C₂₅H₁₇F₂N₅O₅: (Theoretical) C, 59.41; H, 3.39; N, 13.86; (Empirical) C, 59.85; H, 3.49; N, 13.48.

### Example 54

### Preparation of (9)regioisomer-beta-anomer enriched 1-(2'-deoxy-2',2'-difluoro-3',5'-di-O-benzoyl-D-ribofuranosyl)-2,6-(dipivalamido)purine in N,N-dimethylacetamide

2,6-(dipivalamido)purine (0.159 g, 0.5 mmol) was suspended in N,N-dimethylacetamide (1.0 ml) and treated with potassium t-butoxide (0.062 g, 0.55 mmol) and stirred under a nitrogen atmosphere at 25°C to form the potassium salt of 2,6-dipivalamidopurine.

2-deoxy-2,2-difluoro-D-ribofuranosyl-3,5-dibenzoyl-1-α-(p-bromobenzene)sulfonate (0.299 g, 0.5 mmol), in N,N-dimethylacetamide (0.5 ml), was added to the above salt and the entire mixture was reacted for 6 hours at 60°C to form a blocked nucleoside. HPLC analysis confirmed completion of the reaction and indicated a beta to alpha anomeric ratio of 1.9:1 of the titled product.

To isolate the nucleoside product, the reaction mixture was cooled and the solvent removed under vacuum. The residue was diluted with ethyl acetate, washed with sodium bicarbonate, dried over magnesium sulfate and concentrated to an oil. Column chromatography (silica gel, toluene/ethyl acetate 1:1) gave 0.141 g of both alpha and beta nucleoside products at a yield of 28 percent. MS(FD) 679 (M+1).

### Example 55

### Preparation of (9)regioisomer-beta-anomer enriched 1-(2'-deoxy-2',2'-difluoro-3',5'-di-O-benzoyl-D-ribofuranosyl)-2,6-(dipivalamido)purine in acetonitrile

2,6-(dipivalamido)purine (0.159 g, 0.5 mmol) was suspended in acetonitrile (1.5 ml) and treated with potassium t-butoxide (0.062 g, 0.55 mmol) and stirred under a nitrogen atmosphere at 25°C to form the potassium salt of 2,6-(dipivalamido)purine.

2-deoxy-2,2-difluoro-D-ribofuranosyl-3,5-dibenzoyl-1-α-iodide (0.244 g, 0.5 mmol), in acetonitrile (1.5 ml), was added to the above salt and the entire mixture was reacted for 16 hours at 60°C to form a blocked nucleoside. HPLC analysis confirmed completion of the reaction and indicated a beta to alpha anomeric ratio of 2.2:1.

To isolate the nucleoside product, the reaction mixture was diluted with ethyl acetate, the organic layer was washed with sodium bicarbonate, dried over magnesium sulfate separated and concentrated to an oil. Column chromatography (silica gel, toluene/ethyl acetate 1:1) followed by recrystallization gave 0.085 g of the titled product at a yield of 25 percent. MS(FD) 679 (M+1).

### Example 56

### Preparation of beta-anomer enriched 1-(2'-deoxy-2',2'-difluoro-3',5'-di-O-benzoyl-D-ribofuranosyl)-4-(benzylamino)pyrimid-2-one in N,N-dimethylacetamide

N-benzylcytosine (0.099 g, 0.493 mmol) was suspended in N,N-dimethylacetamide (2.0 ml) and treated with sodium hydride (0.0256 g, 0.534 mmol) and stirred under a nitrogen atmosphere at 25°C to form the sodium salt of N-benzylcytosine.

2-deoxy-2,2-difluoro-D-ribofuranosyl-3,5-dibenzoyl-1-α-iodide (0.201 g, 0.411 mmol), in N,N-dimethylacetamide(1.5 ml), was added to the above salt and the entire mixture was reacted for 5 hours at 23°C to form a blocked nucleoside. HPLC analysis confirmed completion of the reaction and indicated a beta to alpha anomeric ratio of 1.9:1.

The reaction solvents were removed under vacuum and the residue was dissolved in ethyl acetate, washed with sodium bicarbonate, dried over magnesium sulfate and concentrated to an oil. Column chromatography (silica gel, toluene/ethyl acetate 9:1) gave 0.015 mg of the titled product at a yield of 6.5 percent. MS(FD) 562 (M+2).

### Example 57

### Preparation of beta-anomer enriched ethyl 1-(2'-deoxy-2',2'-difluoro-3',5'-di-O-benzoyl-D-ribofuranosyl)-1,2,4-triazole-3-carboxylate in N,N-dimethylacetamide

Ethyl 1,2,4-triazole-3-carboxylate (0.723 g, 5.13 mmol) was suspended in N,N-dimethylacetamide (2.5 ml), treated with sodium hydride (0.123 g, 5.13 mmol) and stirred under a nitrogen atmosphere at 25°C to form the sodium salt of the triazole.

2-deoxy-2,2-difluoro-D-ribofuranosyl-3,5-dibenzoyl-1-α-iodide (2.0 g, 4.11 mmol), in N,N-dimethylacetamide(2.5 ml), was added to the above salt and the entire mixture was reacted for 24 hours at 23°C to form a blocked nucleoside. HPLC analysis confirmed completion of the reaction and indicated a beta to alpha anomeric ratio of 3:1.

The crude reaction mixture was purified by removing the solvent under reduced pressure and employing column chromatography (silica gel, toluene/ethyl acetate 9:1). The combined theoretical yield of alpha and beta regioisomers (A and B below) of blocked nucleosides was 67 percent.
A. Ethyl 1-(2'-deoxy-2',2'-difluoro-3',5'-di-O-benzoyl-β -D-ribofuranosyl)-1,2,4-triazole-3-carboxylate (436 mg, 21.2 percent yield). 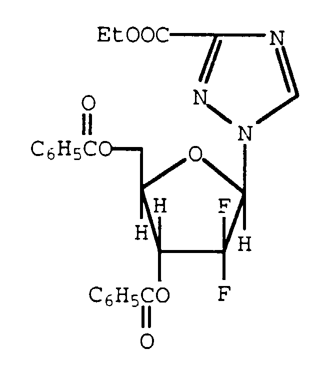
   Recrystallization of "A" from ethyl acetateisooctane provided 267 mg of the pure β-anomer in 13% yield.
B. Ethyl 1-(2'-deoxy-2',2'-difluoro-3',5'-di-O-benzoyl-β -D-ribofuranosyl)-1,2,4-triazole-5-carboxylate (855 mg, 41.5 percent yield).

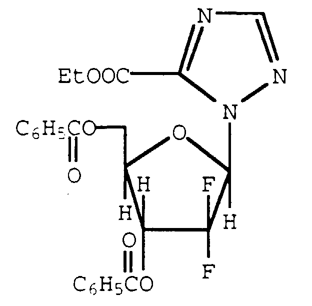

### Example 58

### Preparation of beta-anomer enriched 2-deoxy-2,2-difluoro-D-ribofuranosyl-1-β-(2-amino-6-chloropurine) in dimethylacetamide

To a suspension of 2-amino-6-chloropurine (82.6 mmol, 14.0 g) in dimethylacetamide (900 ml) at 0°C under nitrogen was added powdered potassium hydroxide (99.12 mmol, 5.55 g). The mixture was stirred for 30 minutes to form a solution. 2-deoxy-2,2-difluoro-D-ribofuranosyl-3,5-dibenzoyl-1-α-iodide (82.6 mmol, 40.31 g) in dimethylacetamide (450 ml) was added. The reaction was allowed to warm to room temperature and stirred under nitrogen overnight.

The product was extracted by adding ethyl acetate and brine. The organic layer was washed successively with 1N HCl, saturated sodium bicarbonate solution, H₂O, and brine. The organic layer was then dried over sodium sulfate and evaporated in vacuo.

The crude product was purified with silica gel chromatography to yield a 3:1 beta to alpha anomer ratio of 2-deoxy-2,2-difluoro-D-ribofuranosyl-3,5-dibenzoyl-1-(2-amino-6-chloropurine) ¹H NMR (300 MHz, CD₃OD), δ4.68(m, 2H, 4'-H, 5'a-H), 4.90(m, 1H, 5'b-H), 6.02(m, 1H, 3'-H), 6.29 (m, 1H, 1'-H), 7.53(m, 6H, Bz), 7.92(s, 1H, 8'-H), 8.05(m, 4H, Bz).

The dibenzoyl intermediate (.49 mmol, 260 mg) was deprotected by suspending it in methanol at 0°C and saturating the mixture with anhydrous ammonia. The resulting solution was warmed to room temperature and stirred overnight. The solution was then purged with nitrogen and evaporated. The titled product was then purified by washing with a non-polar solvent such as methylene chloride to remove the benzoate by products. The beta anomer was separated by reversed phase HPLC. ¹H NMR (300MHZ, CD₃OD), ∂ 3.90 (m, 3H, 4'-H,5'-H), 4.58 (m, 1H, 3'-H), 6.27 (dd, 1H, 1'-H), 8.31 (s, 1H, 8-H).

### Preparation 1

### Alpha-anomer enriched 2-deoxy-2,2-difluoro-D-ribofuranosyl-3,5-di-O-benzoyl-1-methanesulfonate

To a solution of 2-deoxy-2,2-difluore-D-ribofuranosyl-3,5-dibenzoate (40 mg) in CD₂Cl₂ (0.5 ml) was added triethylamine (0.025 ml). After stirring at room temperature for 30 minutes the entire mixture was cooled to -78°C then methanesulfonyl chloride (0.01 ml) was added. The reaction temperature was maintained between -78°C and -80°C for 30 minutes then warmed to room temperature. HPLC analysis indicated that the reaction was complete. The anomeric ratio of the titled compound, as determined by ¹⁹F NMR analysis, was 4:1 alpha to beta.

### Preparation 2

### Alpha-anomer 2-deoxy-2,2-difluoro-D-ribofuranosyl-3,5-di-O-benzoyl-1-methanesulfonate

To a solution of 2-deoxy-2,2-difluoro-D-ribofuranosyl-3,5-dibenzoate (60 g, 95% pure) in dichloromethane (600 ml) was added triethylamine (31.5 ml, 1.5 eq.). After stirring at room temperature for 30 minutes the mixture was cooled to -78°C. After 5 minutes, methanesulfonyl chloride (14 ml, 1.2 eq.) in dichloromethane (140 ml) was added to the mixture. The reaction temperature was maintained between -78°C and -80°C under nitrogen for one hour. HPLC analysis indicated that the reaction was complete. The anomeric ratio of the titled compound, as determined by HPLC analysis, was 3.53:1 alpha to beta.

To isolate the titled compound the reaction mixture was washed with water, 1 N HCl solution and 5% sodium bicarbonate solution (300 ml each). The organic layer was separated and dried over anhydrous magnesium sulfate. The titled compound (31.5 g) was obtained in a yield of 46 percent. mp 88-89°C; [α]_{D} (c 1.01, CHCl₃) +84.2°; [α]₃₆₅ₙₘ +302.0°; Elemental Analysis: C₂₀H₁₈O₈SF₂: (Calc.) C 52.63; H 3.98; F 8.33; S 7.02 (456.4) (Actual): C 52.92; H 3.82; F 8.33; S 7.30 ; ¹H NMR (CDCl₃): δ= 3.17 (CH₃), 4.66 and 4.76 (C-5H), 4.84 (C-4H), 5.57 (C-3H), 6.13 (C-1H); ¹³C NMR (CDCl₃) : δ= 40.22 (CH₃), 62.51 (C-5H), 71.03 (C-3H; J_{c,F} = 18.3, 38.5 Hz), 82.75 (C-4H), 99.59 (C-1H; J_{c,F} = 25.5, 48.3 Hz), 122.24 (C-2H; J_{c,F} = 259, 286 Hz).

### Preparation 3

### Alpha-anomer enriched 2-deoxy-2,2-difluoro-D-ribofuranosyl-3,5-di-O-benzoyl-1-methanesulfonate

To an anomeric mixture of 2-deoxy-2,2-difluoro-D-ribofuranosyl-3,5-di-O-benzoyl-1-methanesulfonate (1.0 g, 97% beta-anomer) in acetonitrile (10 ml) was added N,N-dimethylbenzylammonium methanesulfonate (100 mg). The mixture was stirred and heated to reflux. HPLC analysis was used to determine the alpha to beta ratio of the titled product and provided the following:

| Time (hours) | alpha/beta |
|---|---|
| 0 | 1:32 |
| 16 | 1.0:1.4 |
| 24 | 2.3:1.0 |

### Preparation 4

### Alpha-anomer enriched of 2-deoxy-2,2-difluoro-D-ribofuranosyl-3,5-di-O-benzoyl-1-methanesulfonate

To an anomeric mixture of 2-deoxy-2,2-difluoro-D-ribofuranosyl-3,5-di-O-benzoyl-1-methanesulfonate (29.1 g, 50% beta-anomer) in dichloromethane and n-propyl acetate was heated to 90°C to remove the dichloromethane. The mixture was cooled to 50°C-60°C and a mixture of triethylamine (5.33 ml, 0.55 eq) and methanesulfonic acid (2.04 ml, 0.55 eq.) in n-propyl acetate (2 ml) was added. The resulting mixture was heated to 95°C-97°C and stirred. The mixture contained 23.2 g of 2-deoxy-2,2-difluoro-D-ribofuranosyl-3,5-di-O-benzoyl-1-methanesulfonate. HPLC analysis was used to determine the alpha to beta ratio of the titled product and provided the following:

| Time (hours) | alpha/beta |
|---|---|
| 4 | 3:1 |

## Claims

1. A process for preparing a β anomer enriched nucleoside of the formula 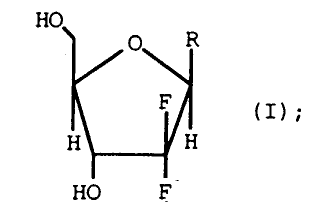 wherein R is a nucleobase selected from the group consisting of 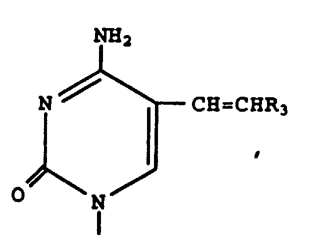 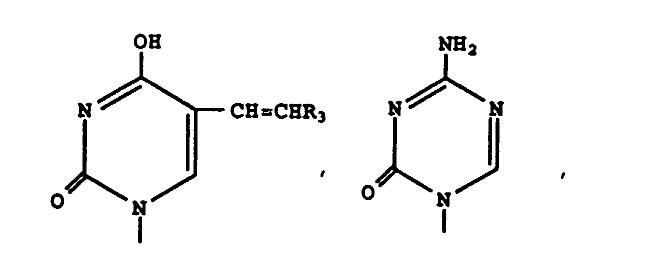 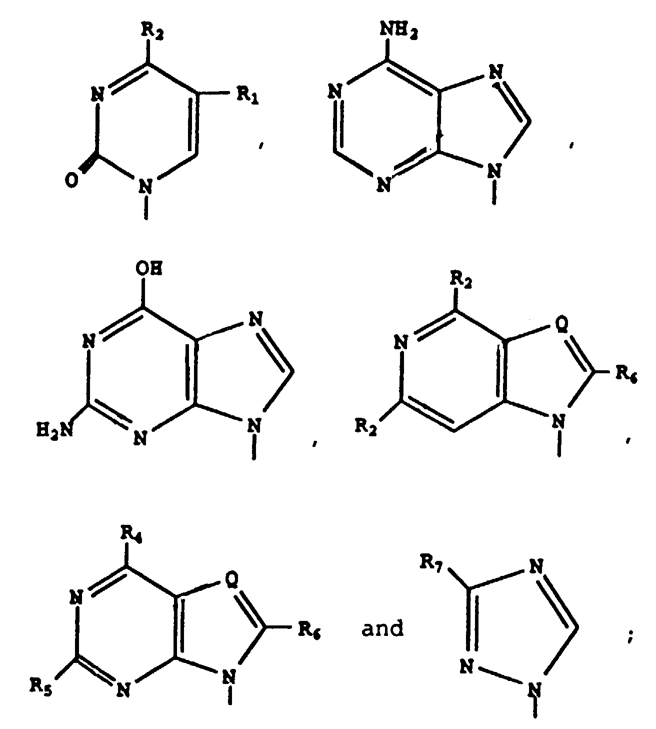 wherein R₁ is selected from the group consisting of hydrogen, alkyl, substituted alkyl and halo; R₂ is selected from the group consisting of hydroxy, halo, azido, primary amino and secondary amino; R₃ is selected from the group consisting of hydrogen, alkyl, and halo; R₇ is selected from the group consisting of hydrogen, halo, cyano, alkyl, alkoxy, alkoxycarbonyl, thioalkyl, thiocarboxamido and carboxamido comprising conducting the S_{N}2 displacement optionally in a suitable solvent of a sulfonyloxy group (Y) from an α anomer enriched carbohydrate of the formula 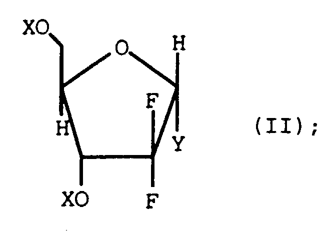 wherein X is independently selected from hydroxy protecting groups; with at least a molar equivalent of a nucleobase (R") selected from the group consisting of 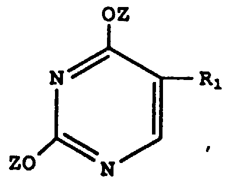 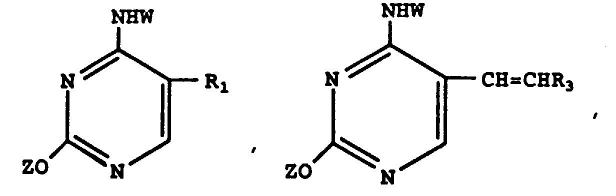 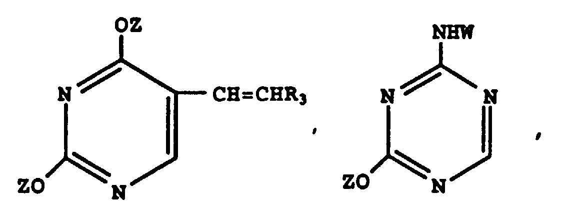 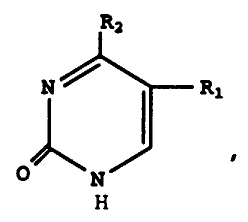 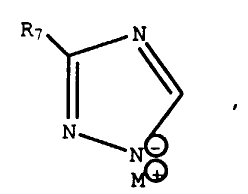 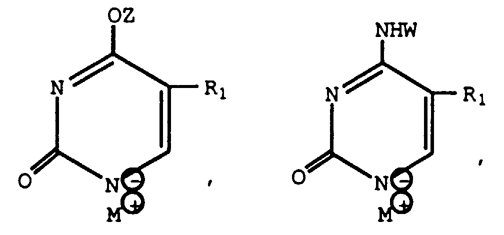 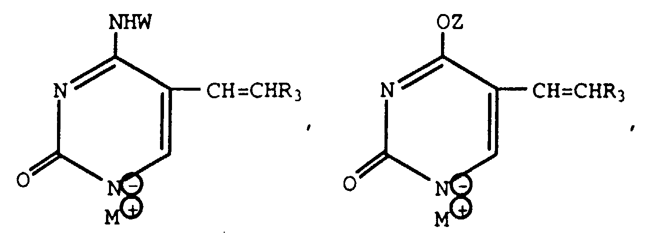 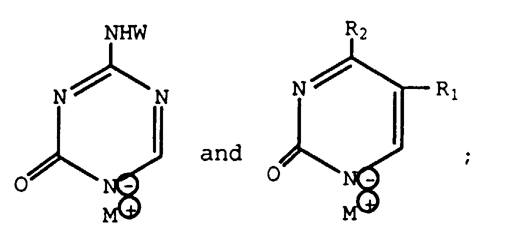 wherein R₁ through R₇ and Q are as defined above and; Z is a hydroxy protecting group; W is an amino protecting group; and M⁺ is a cation; and deblocking to form the compound of the formula (I).

2. The process of Claim 1 wherein R" is selected from the group consisting of 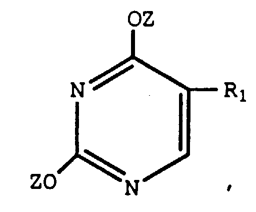 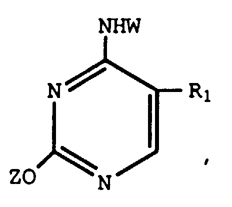 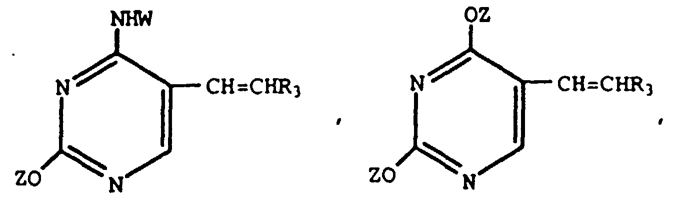 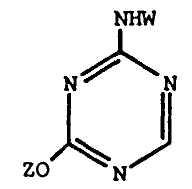 wherein R₁ is selected from the group consisting of hydrogen, alkyl, substituted alkyl and halo; R₃ is selected from the group consisting of hydrogen, alkyl, and halo; Z is a hydroxy protecting group; and W is an amino protecting group; wherein Y is selected from the group consisting of alkylsulfonyloxy, arylsulfonyloxy, substituted alkylsulfonyloxy and substituted arylsulfonyloxy; carried out in solution having a carbohydrate concentration above 20 g/100 ml; and wherein the solvent is a high boiling inert solvent.

3. The process of Claim 1 wherein R" is selected from the group consisting of 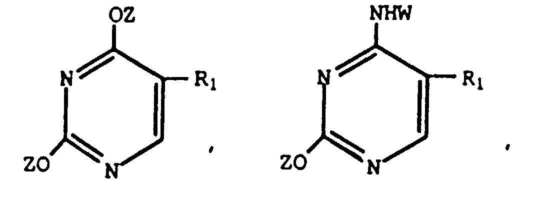 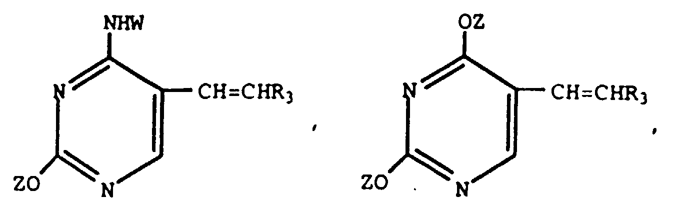 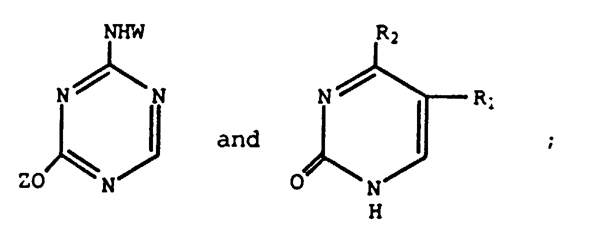 wherein R₁ is selected from the group consisting of hydrogen, alkyl, substituted alkyl and halo; R₂ is selected from the group consisting of hydroxy, halo, azido, primary amino and secondary amino; R₃ is selected from the group consisting of hydrogen, alkyl, and halo; Z is a hydroxy protecting group and W is an amino protecting group; wherein Y is selected from the group consisting of trifluoromethanesulfonyloxy, 1,1,1-trifluoroethanesulfonyloxy, octafluorobutanesulfonyloxy and nonafluorobutanesulfonyloxy; wherein the reaction is effected at a temperature from about -120°C to about 25°C using a low freezing inert solvent.

4. The process of Claim 1 wherein R" is selected from the group consisting of 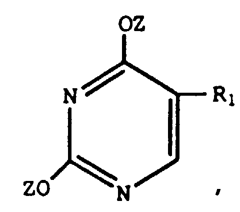 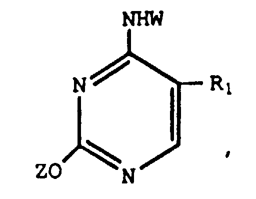 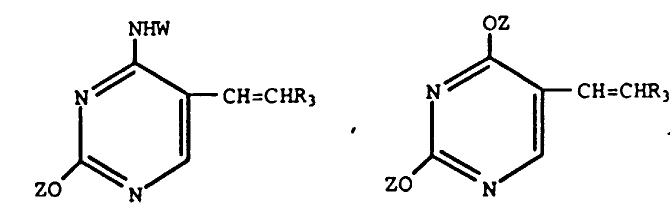 and 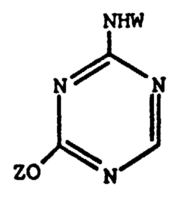 wherein R₁ is selected from the group consisting of hydrogen, alkyl, substituted alkyl and halo; R₃ is selected from the group consisting of hydrogen, alkyl, and halo; Z is a hydroxy protecting group and W is an amino protecting group; carried out in the presence of a catalyst; wherein Y is selected from the group consisting of alkylsulfonyloxy, arylsulfonyloxy, substituted alkylsulfonyloxy and substituted arylsulfonyloxy.

5. The process of Claim 4 wherein the catalyst is selected from highly ionized salts the anion of which is non-nucleophilic and that are substantially soluble in the solvent.

6. The process of Claims 4 or 5 wherein the solvent is selected from polar, non-nucleophilic solvents.

7. The process of Claim 1 wherein R" is 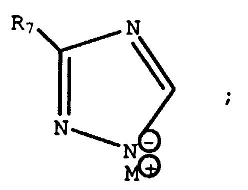 wherein R₇ is selected from the group consisting of hydrogen, halo, cyano, alkyl, alkoxy, alkoxycarbonyl, thioalkyl, thiocarboxamide and carboxamide; and M⁺ is a cation; wherein Y is selected from the group consisting of trifluoromethanesulfonyloxy, 1,1,1-trifluoroethanesulfonyloxy, octafluorobutanesulfonyloxy and nonafluorobutanesulfonyloxy; wherein the solvent is a low freezing inert solvent.

8. The process of Claim 1 carried out in the absence of a solvent and wherein R" is selected from the group consisting of 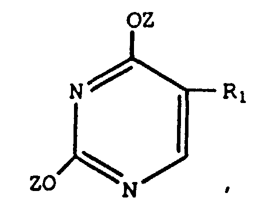 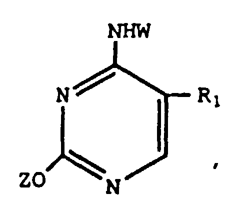 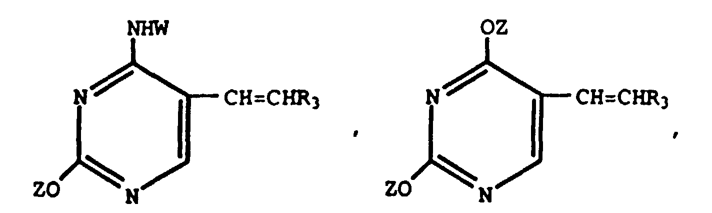 and 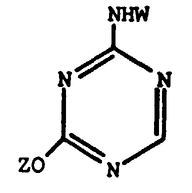 wherein R₁ is selected from the group consisting of hydrogen, alkyl, substituted alkyl and halo; R₃ is selected from the group consisting of hydrogen, alkyl, and halo; Z is a hydroxy protecting group and W is an amino protecting group; wherein Y is selected from the group consisting of alkylsulfonyloxy, arylsulfonyloxy, substituted alkylsulfonyloxy and substituted arylsulfonyloxy.

9. The process of Claim 8 wherein the reaction temperature is from about 100°C to about 160°C.

10. The process of Claim 1 wherein R" is selected from the group consisting of 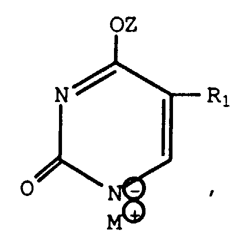 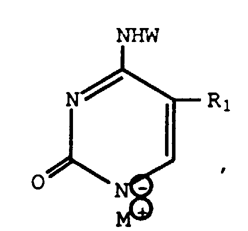 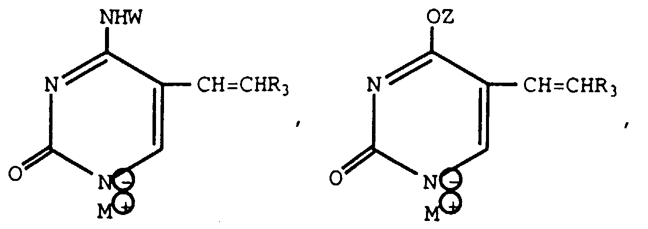 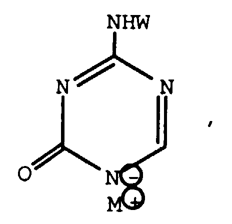 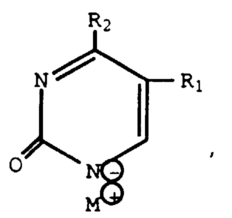 and 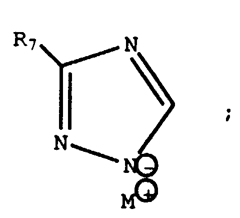 wherein R₁ is selected from the group consisting of hydrogen, alkyl, substituted alkyl and halo; R₂ is selected from the group consisting of hydroxy, halo, azido, primary amino and secondary amino; R₃ is selected from the group consisting of hydrogen, alkyl, and halo; R₇ is selected from the group consisting of hydrogen, halo, cyano, alkyl, alkoxy, alkoxycarbonyl, thioalkyl, thiocarboxamide and carboxamide; Z is a hydroxy protecting group and W is an amino protecting group; wherein Y is selected from the group consisting of alkylsulfonyloxy, arylsulfonyloxy, substituted alkylsulfonyloxy and substituted arylsulfonyloxy.

11. A process according to any one of the preceeding claims for preparing a compound of formula (I) having the structure

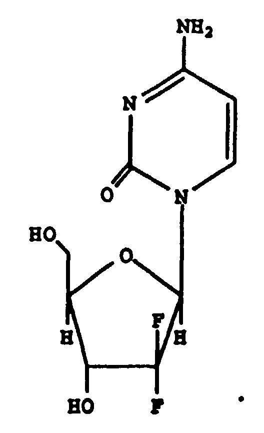

12. A process according to any one of the preceeding claims wherein the blocking group (X) of the compound of formula (II) is benzoyl.

13. A process according to any one of claims 1, 2, 4-6 and 8-12 wherein the sulfonyloxy group (Y) of the compound of formula (II) is methanesulfonyloxy.

14. A process according to any one of claims 1-13 wherein the sulfonyloxy group (Y) of the compound of formula (II) is trifluoromethanesulfonyloxy.

## Patentansprüche

1. Verfahren zur Herstellung eines an β-Anomer angereicherten Nucleosids der Formel 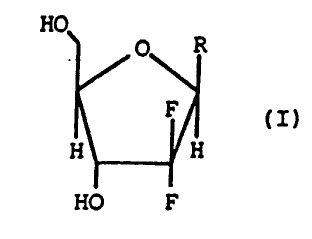 wobei R eine Nucleobase ist, ausgewählt aus der Gruppe bestehend aus 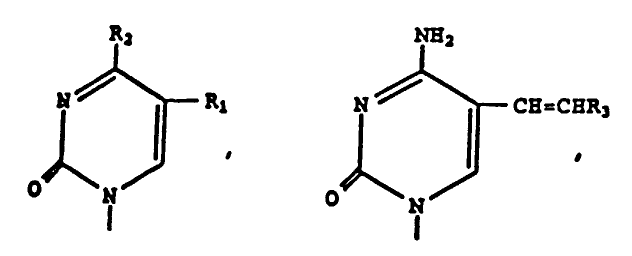 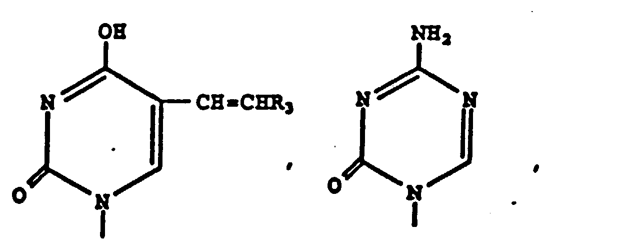 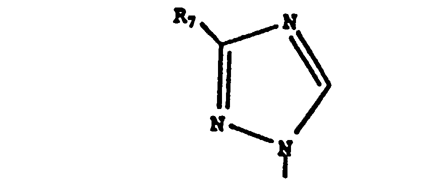 wobei R₁ ausgewählt ist aus der Gruppe bestehend aus Wasserstoff, Alkyl, substituiertem Alkyl und Halogen, R₂ ausgewählt ist aus der Gruppe bestehend aus Hydroxy, Halogen, Azid, primärem Amin und sekundärem Amin, R₃ ausgewählt ist aus der Gruppe bestehend aus Wasserstoff, Alkyl und Halogen, R₇ ausgewählt ist aus der Gruppe bestehend aus Wasserstoff Halogen, Cyan, Alkyl, Alkoxy, Alkoxycarbonyl, Thioalkyl, Thiocarboxamid und Carboxamid,
welches, gegebenenfalls in einem geeigneten Lösungsmittel, Durchführen einer nucleophilen S_{N}2-Substitution einer Sulfonyloxy-Gruppe (Y) aus einem mit einem α-Anomer angereicherten Kohlenhydrat der Formel 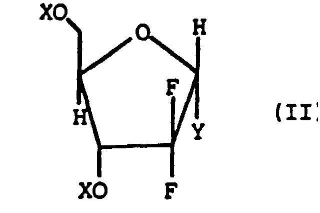 umfaßt, wobei X unabhängig ausgewählt ist unter Hydroxy-Schutzgruppen, mit mindestens einem Moläquivalent einer Nucleobase (R"), ausgewählt aus der Gruppe bestehend aus 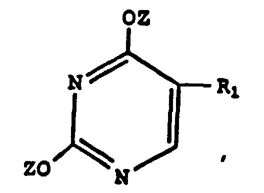 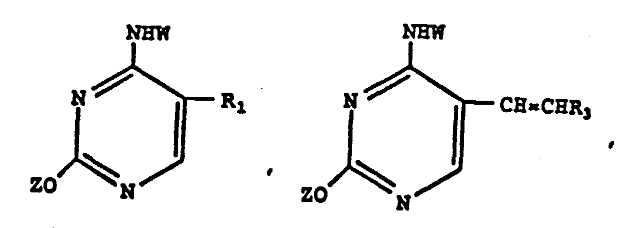 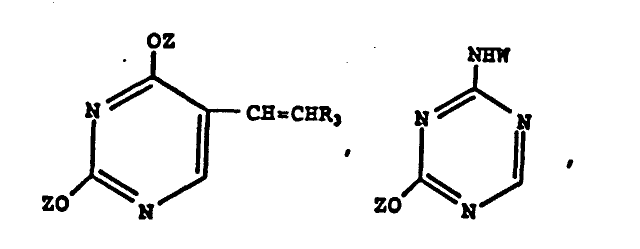 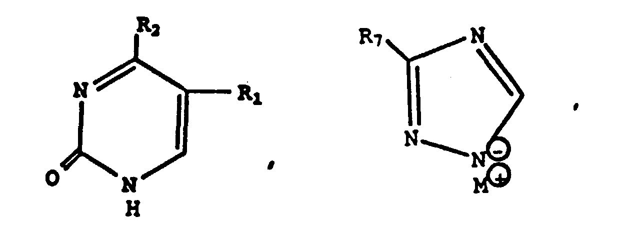 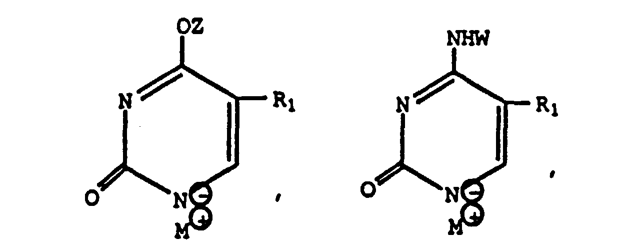 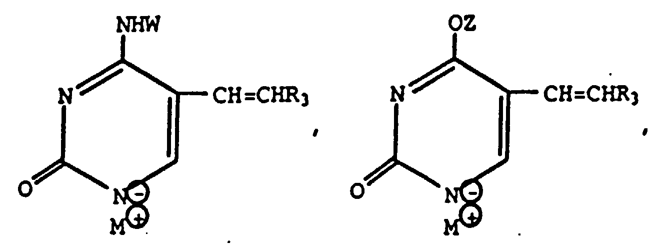 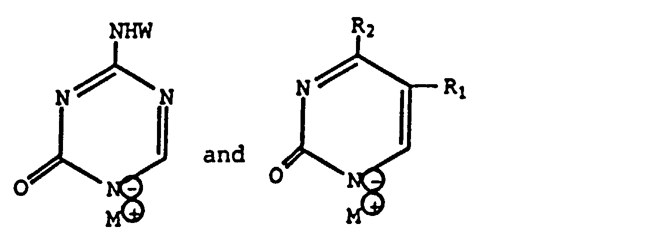 wobei R₁ bis R₇ und Q wie vorstehend definiert sind, Z eine Hydroxy-Schutzgruppe, W eine Amino-Schutzgruppe und M⁺ ein Kation ist, und
Entfernen der Schutzgruppe unter Bildung der Verbindung der Formel (I).

2. Verfahren nach Anspruch 1, wobei R" ausgewählt ist aus der Gruppe bestehend aus 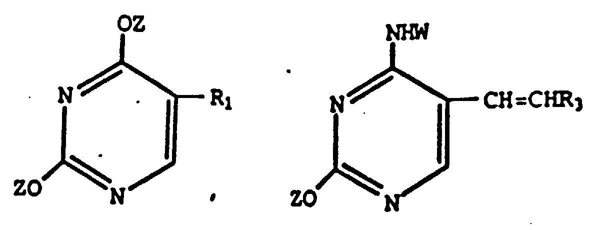 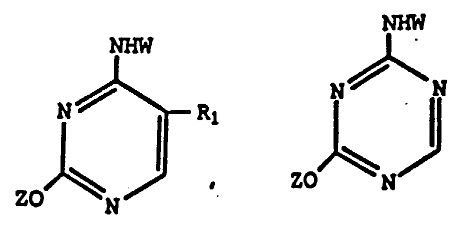 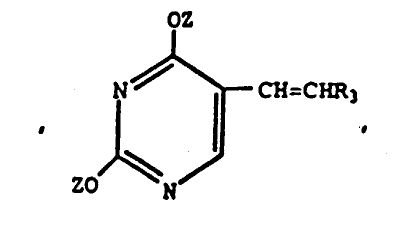 wobei R₁ ausgewählt ist aus der Gruppe bestehend aus Wasserstoff, Alkyl, substituiertem Alkyl und Halogen, R₃ ausgewählt ist aus der Gruppe bestehend aus Wasserstoff, Alkyl und Halogen, Z eine Hydroxy-Schutzgruppe ist und W eine Amino-Schutzgruppe ist, wobei Y ausgewählt ist aus der Gruppe bestehend aus Alkylsulfonyloxy, Arylsulfonyloxy, substituiertem Alkylsulfonyloxy und substituiertem Arylsulfonyloxy, das in einer Lösung mit einer Kohlenhydratkonzentration von 20 g/100ml Lösungsmittel durchgeführt wird und wobei das Lösungsmittel ein inertes, hochsiedendes Lösungsmittel ist.

3. Verfahren nach Anspruch 1, wobei R" ausgewählt ist aus der Gruppe bestehend aus 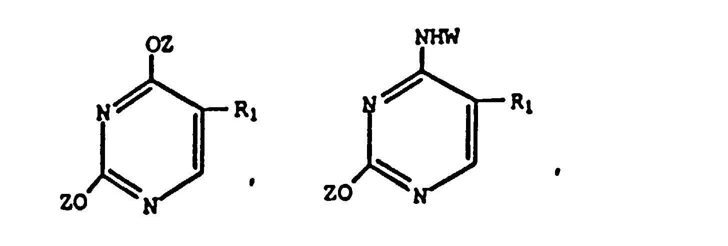 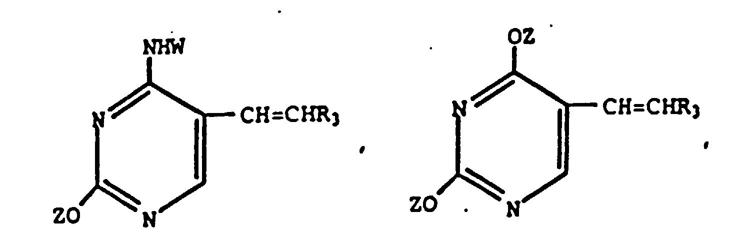 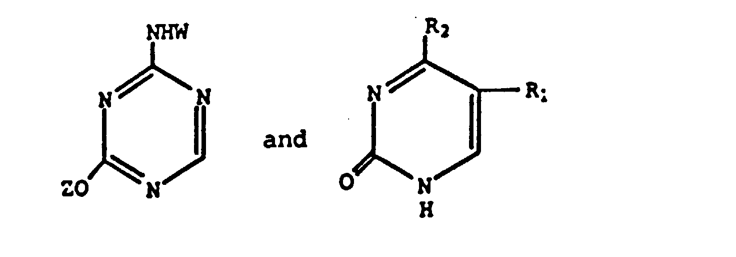 wobei R₁ ausgewählt ist aus der Gruppe bestehend aus Wasserstoff, Alkyl, substituiertem Alkyl und Halogen, R₂ ausgewählt ist aus der Gruppe bestehend aus Hydroxy, Halogen, Azid, primärem Amin und sekundärem Amin, R₃ ausgewählt ist aus der Gruppe bestehend aus Wasserstoff, Alkyl und Halogen, Z eine Hydroxy-Schutzgruppe ist und W eine Amino-Schutzgruppe ist, wobei Y ausgewählt ist aus der Gruppe bestehend aus Trifluormethansulfonyloxy, 1,1,1-Trifluorethansulfonyloxy, Octafluorbutansulfonyloxy und Nonanfluorbutansulfonyloxy,
wobei die Reaktion bei einer Temperatur von etwa -120°C bis etwa 25°C unter Verwendung eines inerten Lösungsmittels mit niedrigem Gefrierpunkt durchgeführt wird.

4. Verfahren nach Anspruch 1, wobei R" ausgewählt ist aus der Gruppe bestehend aus 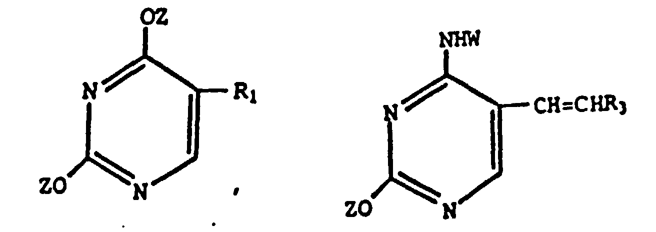 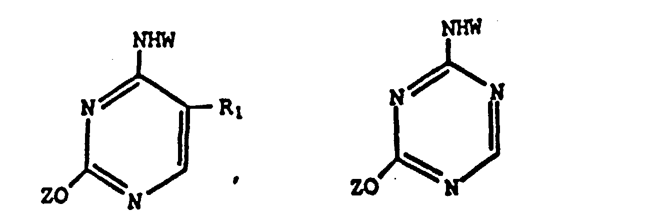 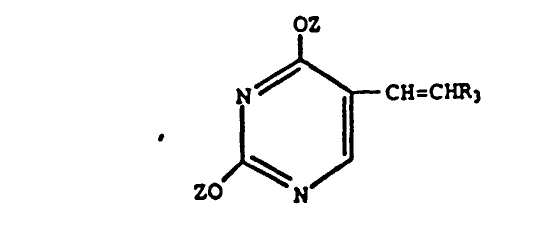 wobei R₁ ausgewählt ist aus der Gruppe bestehend aus Wasserstoff, Alkyl, substituiertem Alkyl und Halogen, R₃ ausgewählt ist aus der Gruppe bestehend aus Wasserstoff, Alkyl und Halogen, Z eine Hydroxy-Schutzgruppe ist und W eine Amino-Schutzgruppe ist, durchgeführt in Anwesenheit eines Katalysators, wobei Y ausgewählt ist aus der Gruppe bestehend aus Alkylsulfonyloxy, Arylsulfonyloxy, substituiertem Alkylsulfonyloxy und substituiertem Arylsulfonyloxy.

5. Verfahren nach Anspruch 4, wobei der Katalysator ausgewählt ist unter stark ionisierten Salzen, deren Anion nicht nucleophil ist und die im Lösungsmittel im wesentlichen löslich sind.

6. Verfahren nach Anspruch 4 oder 5, bei dem das Lösungsmittel ausgewählt ist unter polaren, nicht-nucleophilen Lösungsmitteln.

7. Verfahren nach Anspruch 1, wobei R" 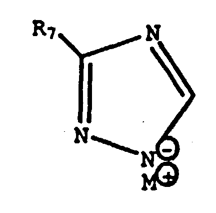 ist, wobei R₇ ausgewählt ist aus der Gruppe bestehend aus Wasserstoff, Halogen, Cyan, Alkyl, Alkoxy, Alkoxycarbonyl. Thioalkyl, Thiocarboxamid und Carboxamid, und M⁺ ein Kation ist, wobei Y ausgewählt ist aus der Gruppe bestehend aus Trifluormethansulfonyloxy, 1,1,1-Trifluorethansulfonyloxy, Octafluorbutansulfonyloxy und Nonafluorbutansulfonyloxy, wobei das Lösungsmittel ein Lösungsmittel mit niedrigem Gefrierpunkt ist.

8. Verfahren nach Anspruch 1, das ohne Lösungsmittel durchgeführt wird und wobei R" ausgewählt ist aus der Gruppe bestehend aus 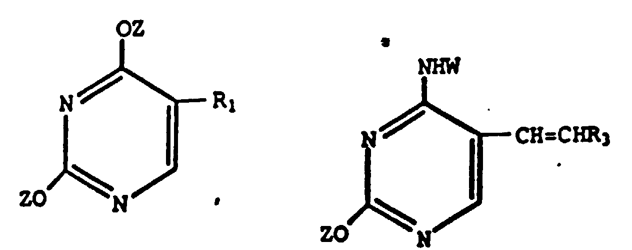 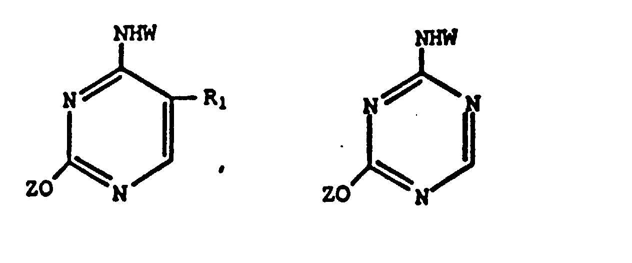 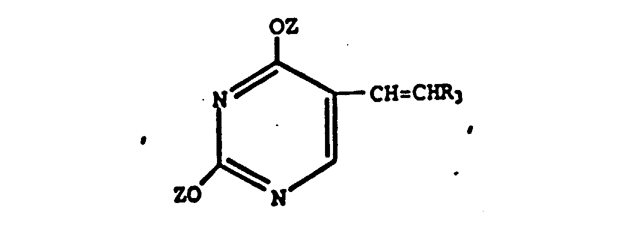 wobei R₁ ausgewählt ist aus der Gruppe bestehend aus Wasserstoff, Alkyl, substituiertem Alkyl und Halogen, R₃ ausgewählt ist aus der Gruppe bestehend aus Wasserstoff, Alkyl und Halogen, Z eine Hydroxy-Schutzgruppe ist und W eine Amino-Schutzgruppe ist, wobei Y ausgewählt ist aus der Gruppe bestehend aus Alkylsulfonyloxy, Arylsulfonyloxy, substituiertem Alkylsulfonyloxy und substituiertem Arylsulfonyloxy.

9. Verfahren nach Anspruch 8, wobei die Reaktionstemperatur von etwa 100°C bis etwa 160°C beträgt.

10. Verfahren nach Anspruch 1, wobei R" ausgewählt ist aus der Gruppe bestehend aus 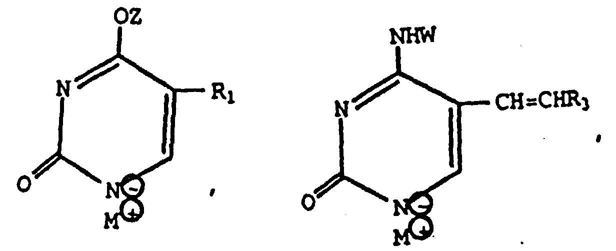 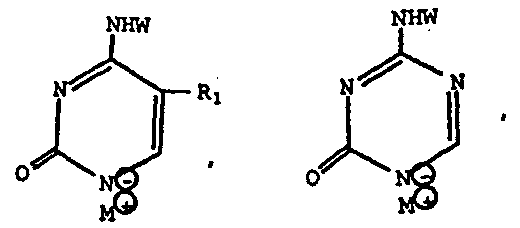 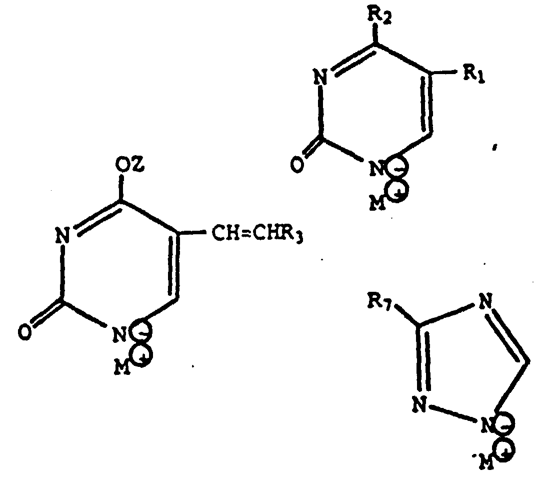 wobei R₁ ausgewählt ist aus der Gruppe bestehend aus Wasserstoff, Alkyl, substituiertem Alkyl und Halogen, R₂ ausgewählt ist aus der Gruppe bestehend aus Hydroxy, Halogen, Azid, primärem Amin und sekundärem Amin, R₃ ausgewählt ist aus der Gruppe bestehend aus Wasserstoff, Alkyl und Halogen, R₇ ausgewählt ist aus der Gruppe bestehend aus Wasserstoff, Halogen, Cyan, Alkyl, Alkoxy, Alkoxycarbonyl, Thioalkyl, Thiocarboxamid und Carboxamid, Z eine Hydroxy-Schutzgruppe ist und W eine Amino-Schutzgruppe ist, wobei Y ausgewählt ist aus der Gruppe bestehend aus Alkylsulfonyloxy, Arylsulfonyloxy, substituiertem Alkylsulfonyloxy und substituiertem Arylsulfonyloxy.

11. Verfahren nach einem der vorhergehenden Ansprüche, zur Herstellung einer Verbindung der Formel (I) welche die folgende Struktur aufweist

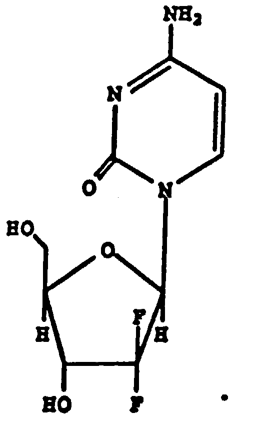

12. Verfahren nach einem der vorhergehenden Ansprüche , wobei die Schutzgruppe (X) der Verbindung der Formel (II) Benzoyl ist.

13. Verfahren nach einem der Ansprüche 1, 2, 4 bis 6 und 8 bis 12, wobei die Sulfonyloxygruppe (Y) der Verbindung der Formel (II) Methansulfonyloxy ist.

14. Verfahren nach einem der Ansprüche 1 - 13, wobei die Sulfonyloxygruppe (Y) der Verbindung der Formel (II) Trifluormethansulfonyloxy ist.

## Revendications

1. Procédé pour préparer un nucléoside enrichi en anomère β répondant à la formule 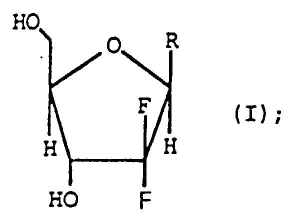 dans laquelle R représente une base nucléique choisie parmi le groupe constitué par 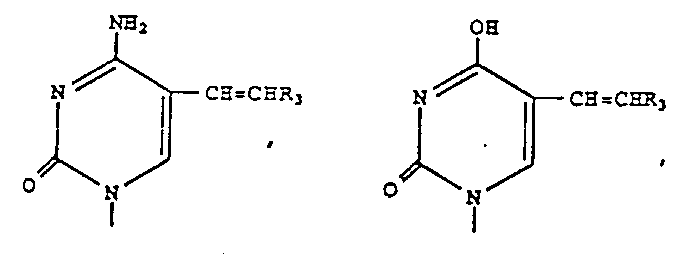 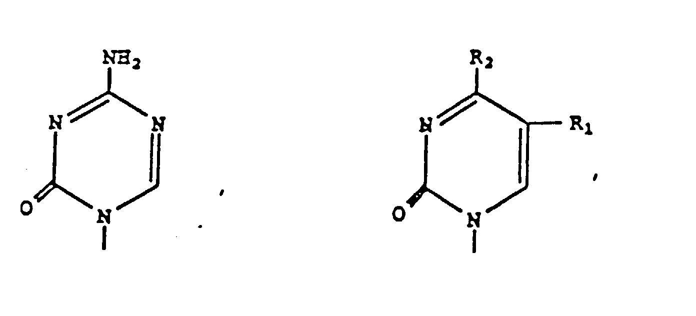 et 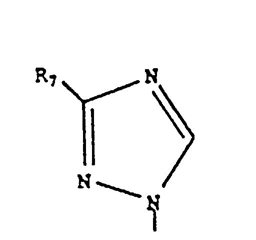 où R₁ est choisi parmi le groupe constitué par un atome d'hydrogène, un groupe alkyle, un groupe alkyle substitué et un atome d'halogène; R₂ est choisi parmi le groupe constitué par un groupe hydroxyle, un atome d'halogène, un groupe azido, un groupe amino primaire et un groupe amino secondaire; R₃ est choisi parmi le groupe constitué par un atome d'hydrogène, un groupe alkyle, un atome d'halogène; R₇ est choisi parmi le groupe constitué par un atome d'hydrogène, un atome d'halogène, un groupe cyano, un groupe alkyle, un groupe alcoxy, un groupe alcoxycarbonyle, un groupe thioalkyle, un groupe thiocarboxamido et un groupe carboxamido; comprenant le fait de réaliser la substitution de type S_{N}2, le cas échéant dans un solvant approprié, d'un groupe sulfonyloxy (Y) à partir d'un glucide enrichi en anomère α répondant à la formule 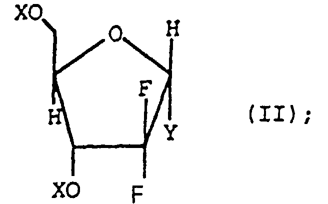 dans laquelle les radicaux X sont choisis, indépendamment l'un de l'autre, parmi les groupes protecteurs du groupe hydroxyle; avec au moins un équivalent molaire d'une base nucléique (R") choisie parmi le groupe constitué par 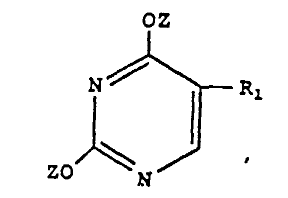 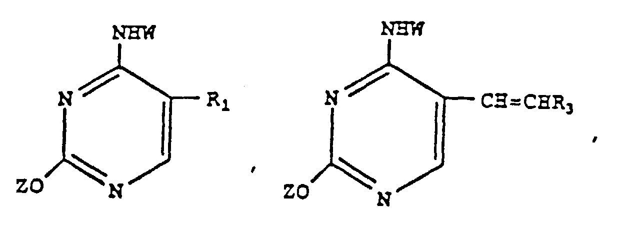 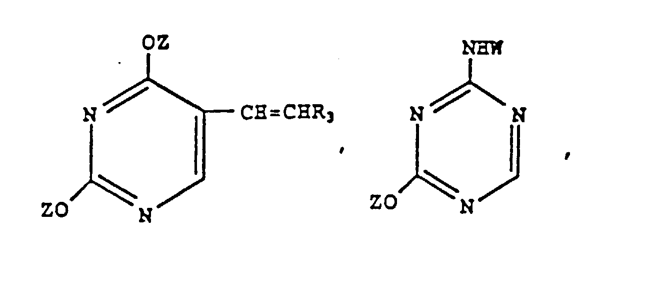 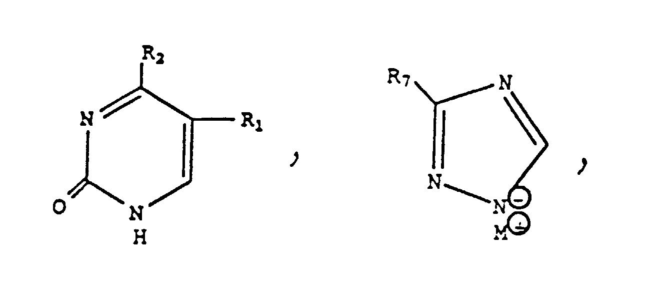 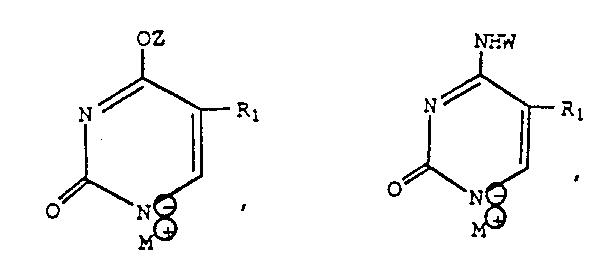 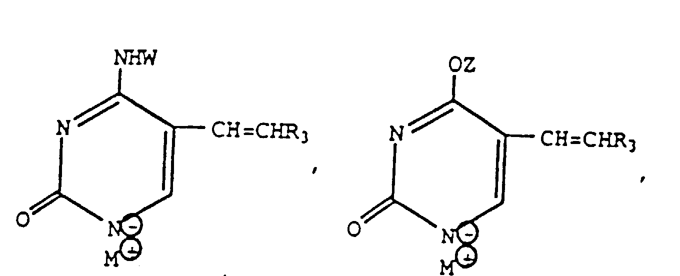 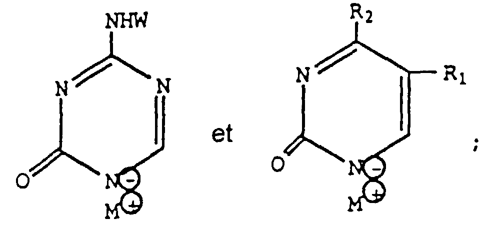 où R₁ à R₇ et Q sont tels que définis ci-dessus; Z représente un groupe protecteur du groupe hydroxyle; W représente un groupe protecteur du groupe amino et M⁺ représente un cation; et un déblocage pour obtenir le composé de formule (I).

2. Procédé selon la revendication 1, dans lequel R" est choisi parmi le groupe constitué par 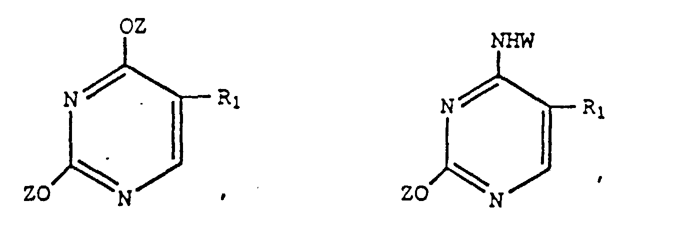 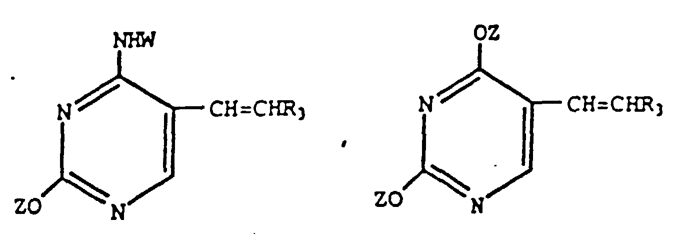 et 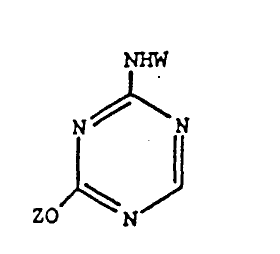 où R₁ est choisi parmi le groupe constitué par un atome d'hydrogène, un groupe alkyle, un groupe alkyle substitué et un atome d'halogène; R₃ est choisi parmi le groupe constitué par un atome d'hydrogène, un groupe alkyle et un atome d'halogène; Z représente un groupe protecteur du groupe hydroxyle et W représente un groupe protecteur du groupe amino; Y est choisi parmi le groupe constitué par un groupe alkylsulfonyloxy, un groupe arylsulfonyloxy, un groupe alkylsulfonyloxy substitué et un groupe arylsulfonyloxy substitué; mis en oeuvre dans une solution possédant une concentration de glucide supérieure à 20 g/100 ml de solvant; et dans lequel le solvant est un solvant inerte à point d'ébullition élevé.

3. Procédé selon la revendication 1, dans lequel R" est choisi parmi le groupe constitué par 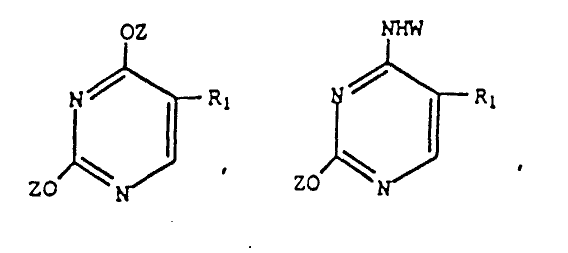 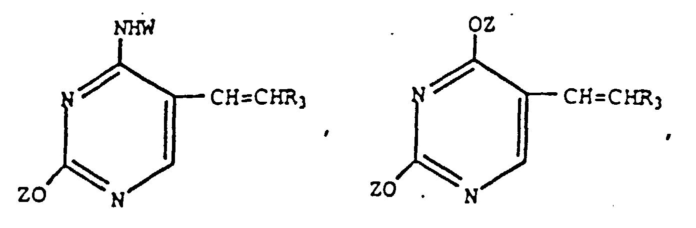 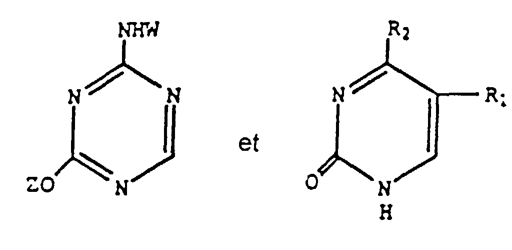 où R₁ est choisi parmi le groupe constitué par un atome d'hydrogène, un groupe alkyle, un groupe alkyle substitué et un atome d'halogène; R₂ est choisi parmi le groupe constitué par un groupe hydroxyle, un atome d'halogène, un groupe azido, un groupe amino primaire et un groupe amino secondaire; R₃ est choisi parmi le groupe constitué par un atome d'hydrogène, un groupe alkyle et un atome d'halogène; Z représente un groupe protecteur du groupe hydroxyle et W représente un groupe protecteur du groupe amino; Y est choisi parmi le groupe constitué par un groupe trifluorométhanesulfonyloxy, un groupe 1,1,1,trifluoro-éthanesulfonyloxy, un groupe octafluorobutanesulfonyloxy et un groupe nonafluorobutanesulfonyloxy; dans lequel on effectue la réaction à une température d'environ -120°C à environ 25°C en utilisant un solvant inerte à bas point de congélation.

4. Procédé selon la revendication 1, dans lequel R" est choisi parmi le groupe constitué par 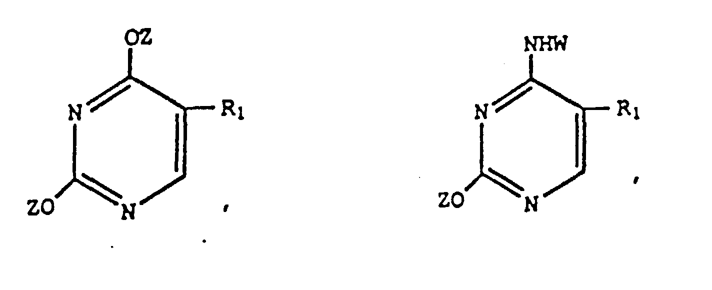 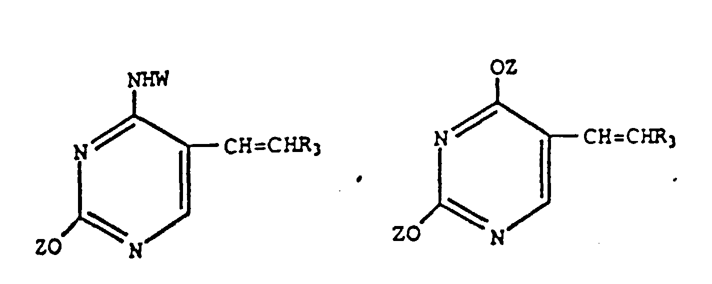 et 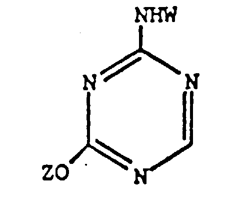 où R₁ est choisi parmi le groupe constitué par un atome d'hydrogène, un groupe alkyle, un groupe alkyle substitué et un atome d'halogène; R₃ est choisi parmi le groupe constitué par un atome d'hydrogène, un groupe alkyle et un atome d'halogène; Z représente un groupe protecteur du groupe hydroxyle et W représente un groupe protecteur du groupe amino; que l'on effectue en présence d'un catalyseur et dans lequel Y est choisi parmi le groupe constitué par un groupe alkylsulfonyloxy, un groupe arylsulfonyloxy, un groupe alkylsulfonyloxy substitué et un groupe arylsulfonyloxy substitué.

5. Procédé selon la revendication 4, dans lequel le catalyseur est choisi parmi des sels fortement ionisés dont l'anion est de type nucléophile et qui sont essentiellement solubles dans les solvants.

6. Procédé selon la revendication 4 ou 5, dans lequel le solvant est choisi parmi des solvants polaires non nucléophiles.

7. Procédé selon la revendication 1, dans lequel R" répond à la formule 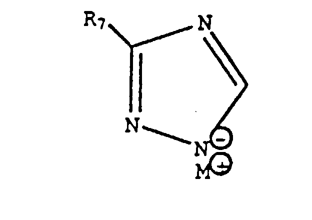 dans laquelle R₇ est choisi parmi le groupe constitué par un atome d'hydrogène, un atome d'halogène, un groupe cyano, un groupe alkyle, un groupe alcoxy, un groupe alcoxycarbonyle, un groupe thioalkyle, un groupe thiocarboxamide et un groupe carboxamide; et M⁺ représente un cation; dans lequel Y est choisi parmi le groupe constitué par un groupe trifluorométhanesulfonyloxy, un groupe 1,1,1,-trifluoro-éthanesulfonyloxy, un groupe octafluorobutanesulfonyloxy et un groupe nonafluorobutanesulfonyloxy; dans lequel le solvant est un solvant inerte à bas point de congélation.

8. Procédé selon la revendication 1, mis en oeuvre en l'absence d'un solvant et dans lequel R" est choisi parmi le groupe constitué par 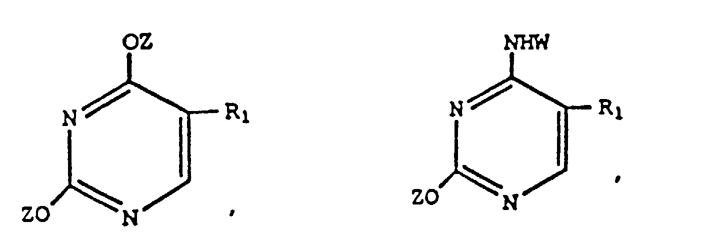 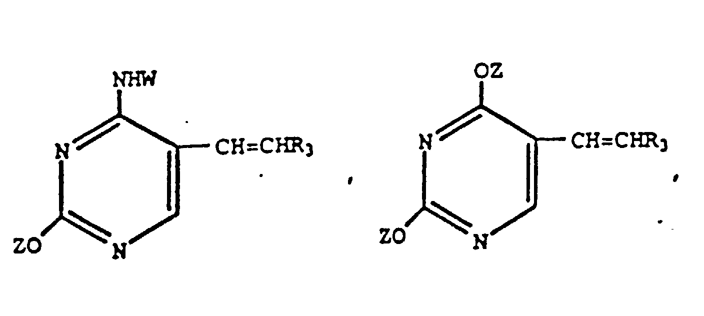 et 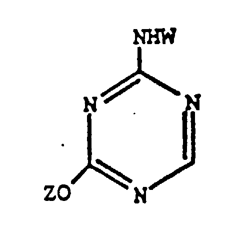 où R₁ est choisi parmi le groupe constitué par un atome d'hydrogène, un groupe alkyle, un groupe alkyle substitué et un atome d'halogène; R₃ est choisi parmi le groupe constitué par un atome d'hydrogène, un groupe alkyle et un atome d'halogène; Z représente un groupe protecteur du groupe hydroxyle et W représente un groupe protecteur du groupe amino; dans lequel Y est choisi parmi le groupe constitué par un groupe alkylsulfonyloxy, un groupe arylsulfonyloxy, un groupe alkylsulfonyloxy substitué et un groupe arylsulfonyloxy substitué.

9. Procédé selon la revendication 8, dans lequel la température réactionnelle est d'environ 100°C à environ 160°C.

10. Procédé selon la revendication 1, dans lequel R" est choisi parmi le groupe constitué par 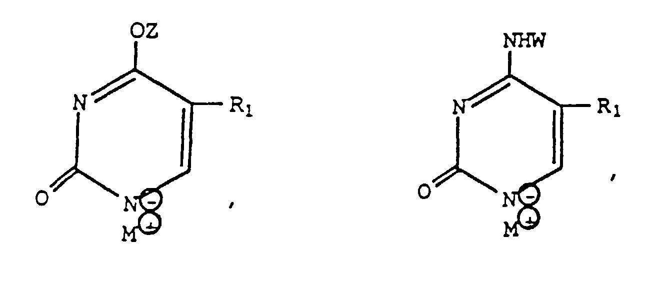 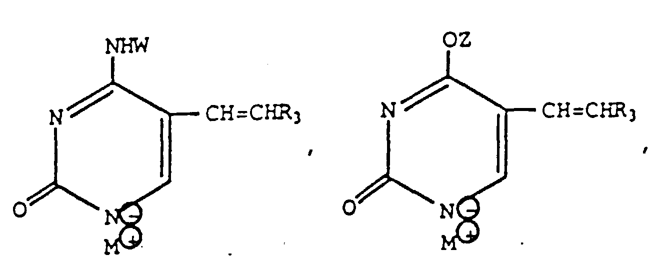 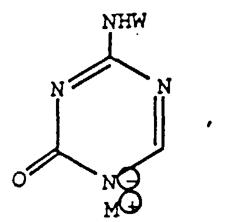 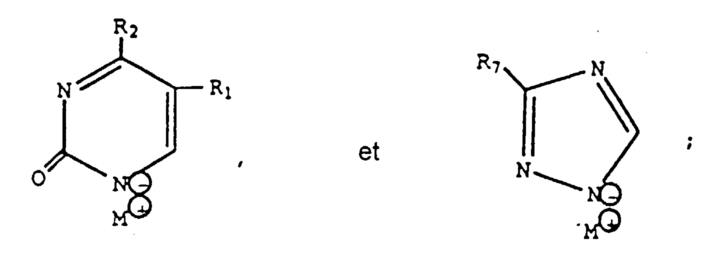 où R₁ est choisi parmi le groupe constitué par un atome d'hydrogène, un groupe alkyle, un groupe alkyle substitué et un atome d'halogène; R₂ est choisi parmi le groupe constitué par un groupe hydroxyle, un atome d'halogène, un groupe azido, un groupe amino primaire et un groupe amino secondaire; R₃ est choisi parmi le groupe constitué par un atome d'hydrogène, un groupe alkyle et un atome d'halogène; R₇ est choisi parmi le groupe constitué par un atome d'hydrogène, un atome d'halogène, un groupe cyano, un groupe alkyle, un groupe alcoxy, un groupe alcoxycarbonyle, un groupe thioalkyle, un groupe thiocarboxamide et un groupe carboxamide; Z représente un groupe protecteur du groupe hydroxyle et W représente un groupe protecteur du groupe amino; dans lequel Y est choisi parmi le groupe constitué par un groupe alkylsulfonyloxy, un groupe arylsulfonyloxy, un groupe alkylsulfonyloxy substitué et un groupe arylsulfonyloxy substitué.

11. Procédé selon l'une quelconque des revendications précédentes pour préparer un composé de formule (I) répondant à la structure

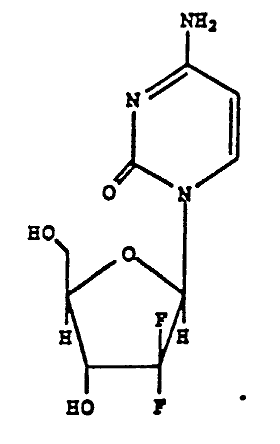

12. Procédé selon l'une quelconque des revendications précédentes, dans lequel le groupe de blocage (X) du composé de formule (II) est un groupe benzoyle.

13. Procédé selon l'une quelconque des revendications 1, 2, 4-6 et 8-12, dans lequel le groupe sulfonyloxy (Y) du composé de formule (II) est un groupe méthanesulfonyloxy.

14. Procédé selon l'une quelconque des revendications 1-13, dans lequel le groupe sulfonyloxy (Y) du composé de formule (II) est un groupe trifluorométhanesulfonyloxy.
